(19) Europäisches Patentamt

European Patent Office

Office européen des brevets

(11) **EP 4 329 848 B1**

(12) **EUROPEAN PATENT SPECIFICATION**

(45) Date of publication and mention
of the grant of the patent:
**14.01.2026 Bulletin 2026/03**

(21) Application number: **22722579.4**

(22) Date of filing: **29.04.2022**

(51) International Patent Classification (IPC):
*A61M 16/00* (2006.01)    *A61B 5/087* (2006.01)
*A61B 5/00* (2006.01)

(52) Cooperative Patent Classification (CPC):
**A61M 16/024; A61B 5/087; A61B 5/4812;**
**A61B 5/4818; A61B 5/4836;** A61B 5/0077;
A61B 5/0086; A61B 5/02055; A61B 5/02416;
A61B 5/0816; A61B 5/082; A61B 5/1118;
A61B 5/318; A61B 5/369; A61B 5/7475;    (Cont.)

(86) International application number:
**PCT/IB2022/053977**

(87) International publication number:
**WO 2022/229910 (03.11.2022 Gazette 2022/44)**

(54) **SYSTEMS FOR MODIFYING PRESSURE SETTINGS OF A RESPIRATORY THERAPY SYSTEM**

SYSTEME ZUR MODIFIZIERUNG VON DRUCKEINSTELLUNGEN EINES
ATEMTHERAPIESYSTEMS

SYSTÈMES MODIFICATION DES RÉGLAGES DE PRESSION D'UN SYSTÈME DE THÉRAPIE
RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.04.2021   US 202163181829 P**

(43) Date of publication of application:
**06.03.2024 Bulletin 2024/10**

(73) Proprietor: **ResMed Sensor Technologies Limited
Sandyford D18 T6T7
Dublin (IE)**

(72) Inventors:
• **LYON, Graeme Alexander
  Dublin, D18 T6T7 (IE)**
• **CHAH, Ehsan
  Dublin, D18 T6T7 (IE)**
• **TIRON, Roxana
  Dublin, D18 T6T7 (IE)**

(74) Representative: **Mathys & Squire
Theatinerstraße 7
80333 München (DE)**

(56) References cited:
US-A- 5 245 995        US-A1- 2008 097 234
US-A1- 2015 250 963    US-A1- 2017 274 165

(52) Cooperative Patent Classification (CPC): (Cont.)
A61M 16/0063; A61M 2016/0015; A61M 2016/0027;
A61M 2016/003; A61M 2230/40

**Description**

**CROSS-REFERENCE TO RELATED APPLICATIONS**

This application claims the benefit of, and priority to, U.S. Provisional Patent Application No. 63/181,829 filed on April 29, 2021.

**TECHNICAL FIELD**

The present disclosure relates generally to systems and methods for modifying pressure settings of a respiratory therapy system, and more particularly, to systems and methods for modifying pressure settings of a respiratory therapy system for a subsequent portion of a current sleep session or a portion of a subsequent sleep session, based on events experienced during a current sleep session.

**BACKGROUND**

Many individuals suffer from sleep-related and/or respiratory disorders such as, for example, Sleep-Disordered Breathing (SDB), which can include Obstructive Sleep Apnea (OSA), Central Sleep Apnea (CSA), other types of apneas such as mixed apneas and hypopneas, and Respiratory Effort Related Arousal (RERA). These individuals may also suffer from other health conditions (which may be referred to as comorbidities), such as insomnia (characterized by, for example, difficult in initiating sleep, frequent or prolonged awakenings after initially falling asleep, and/or an early awakening with an inability to return to sleep), Periodic Limb Movement Disorder (PLMD), Restless Leg Syndrome (RLS), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, and chest wall disorders.

These individuals are often treated using a respiratory therapy system (e.g., a continuous positive airway pressure (CPAP) system), which delivers pressurized air to aid in preventing the individual's airway from narrowing or collapsing during sleep. However, it can be difficult to effectively manage the pressure of the pressurized air and minimize the amount of disruptions or events the user may experience as they sleep. Examples of respiratory therapy systems can be found in US2008/097234 A1, US2015/250963 A1, US2017/274165 A1 or US5245995 A. Thus, it would be advantageous to improve the management of the pressurized air supplied to the user's airway. The present disclosure is directed to solving these and other problems.

**SUMMARY**

The invention is defined by the appended claims. According to some implementations of the present disclosure, a system comprises a respiratory therapy system, a memory, and a control system. The respiratory therapy system is configured to supply pressurized air at a first pressure of a predetermined range of pressures to a user via the respiratory therapy system during a current sleep session. The predetermined range of pressures further includes at least a second pressure greater than the first pressure. The memory stores machine-readable instructions. The control system includes one or more processors configured to execute the machine-readable instructions to implement a method. The method includes determining a number of events experienced by the user during a first portion of the current sleep session while the pressurized air is supplied at the first pressure, determining a type of each event experienced by the user during the first portion of the current sleep session, or both. The method further includes, in response to the number of events, the type of each event, or both experienced during the first portion of the current sleep session satisfying a threshold, performing one or more modifications. The modifications can include (i) modifying the first pressure to be a modified first pressure for a subsequent sleep session portion of the user, such that in response to a determination that at a beginning of the subsequent sleep session portion, the pressurized air is supplied to the user via the respiratory therapy system at the modified first pressure, (ii) modifying a difference in pressure between the first pressure and the second pressure of the predetermined range of pressures for the subsequent sleep session portion, (iii) modifying a rate of change from the first pressure to the second pressure of the predetermined range of pressures for the subsequent sleep session portion, or (iv) any combination of (i)-(iii).

According to some implementations of the present disclosure, a system comprises a respiratory therapy system, a memory, and a control system. The respiratory therapy system is configured to supply pressurized air at a first pressure of a predetermined range of pressures to a user via the respiratory therapy system during a current sleep session. The predetermined range of pressures further includes at least a second pressure greater than the first pressure. The memory stores machine-readable instructions. The control system includes one or more processors configured to execute the machine-readable instructions to implement a method. The method includes determining the number of events experienced by the user during a first portion of the current sleep session while the pressurized air is supplied at the first pressure, determining a type of each event experienced by the user during the first portion of the current sleep session, or both. The method further includes, in response to the number of events, the type of each event, or both experienced during the first portion of the current sleep session satisfying a threshold, performing one or more modifications. The modifications can include (i) modifying the first pressure to be a modified first pressure for a subsequent sleep session portion of the user, such that at a beginning of the subsequent sleep session portion, the pressurized air is supplied to the user via the respiratory therapy system at the modified first pressure, (ii) modifying a difference in pressure between the first pressure and the second pressure of the predetermined range of pressures for the subsequent sleep session portion, (iii) modifying a rate of change from the first pressure to the second pressure of the predetermined range of pressures for the subsequent sleep session portion, or (iv) any combination of (i)-(iii).

According to some implementations of the present disclosure, a method of modifying pressure settings of a respiratory therapy system comprises supplying pressurized air at a first pressure of a predetermined range of pressures to a user via the respiratory therapy system during a current sleep session. The predetermined range of pressures includes at least a second pressure greater than the first pressure. The method further includes determining the number of events experienced by the user during a first portion of the current sleep session while the pressurized air is supplied at the first pressure, determining a type of each event experienced by the user during the first portion of the current sleep session, or both. The method further includes, in response to the number of events, the type of each event, or both experienced during the first portion of the current sleep session satisfying a threshold, performing one or more modifications. The modifications can include (i) modifying the first pressure to be a modified first pressure for a subsequent sleep session portion of the user, such that at a beginning of the subsequent sleep session portion, the pressurized air is supplied to the user via the respiratory therapy system at the modified first pressure, (ii) modifying a difference in pressure between the first pressure and the second pressure of the predetermined range of pressures for the subsequent sleep session portion, (iii) modifying a rate of change from the first pressure to the second pressure of the predetermined range of pressures for the subsequent sleep session portion, or (iv) any combination of (i)-(iii).

According to some implementations of the present disclosure, a method of modifying pressure settings of a respiratory therapy system comprises supplying pressurized air at a plurality of pressures of a predetermined range of pressures to a user via the respiratory therapy system during a current sleep session. The predetermined range of pressures includes at least a first pressure and a second pressure greater than the first pressure. The method further includes determining a total amount of time during the current sleep session that the pressure of the pressurized air is greater than a modification threshold pressure. The method further includes, in response to the determined total amount of time satisfying a modification threshold amount of time, performing one or more modifications. The modifications can include (i) modifying the first pressure to be a modified first pressure for a subsequent sleep session portion of the user, such that at a beginning of the subsequent sleep session portion, the pressurized air is supplied to the user via the respiratory therapy system at the modified first pressure, (ii) modifying a difference in pressure between the first pressure and the second pressure of the predetermined range of pressures for the subsequent sleep session portion, (iii) modifying a rate of change from the first pressure to the second pressure of the predetermined range of pressures for the subsequent sleep session portion, or (iv) any combination of (i)-(iii).

The above summary is not intended to represent each implementation or every aspect of the present disclosure. Additional features and benefits of the present disclosure are apparent from the detailed description and figures set forth below.

**BRIEF DESCRIPTION OF THE DRAWINGS**

FIG. 1 is a functional block diagram of a respiratory therapy system, according to some implementations of the present disclosure;

FIG. 2 is a perspective view of the respiratory therapy system of FIG. 1, a user of the respiratory therapy system, and a bed partner of the user, according to some implementations of the present disclosure;

FIG. 3 illustrates an exemplary timeline for a sleep session, according to some implementations of the present disclosure;

FIG. 4 illustrates an exemplary hypnogram associated with the sleep session of FIG. 3, according to some implementations of the present disclosure;

FIG. 5 is a process flow diagram for a first method of modifying pressure settings of a respiratory therapy system, according to some implementations of the present disclosure;

FIG. 6A is a pressure versus time plot of a first technique for modifying the pressure settings of the respiratory therapy system during a subsequent portion of a sleep session, according to some implementations of the present disclosure;

FIG. 6B is a pressure versus time plot of a second technique for modifying the pressure settings of the respiratory therapy system during a subsequent portion of a sleep session, according to some implementations of the present disclosure;

FIG. 6C is a pressure versus time plot of a third technique for modifying the pressure settings of the respiratory therapy system during a subsequent portion of a sleep session, according to some implementations of the present disclosure;

FIG. 6D is a pressure versus time plot of a fourth technique for modifying the pressure settings of the respiratory therapy system during a subsequent portion of a sleep session, according to some implementations of the present disclosure; and

FIG. 7 is a process flow diagram for a second method of modifying pressure settings of a respiratory therapy system, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, equivalents, and alternatives falling within the scope of the present disclosure as defined by the appended claims.

## DETAILED DESCRIPTION

The present disclosure is described with reference to the attached figures, where like reference numerals are used throughout the figures to designate similar or equivalent elements. The figures are not drawn to scale, and are provided merely to illustrate the instant disclosure. Several aspects of the disclosure are described below with reference to example applications for illustration.

Many individuals suffer from sleep-related and/or respiratory-related disorders. Examples of sleep-related and/or respiratory disorders include Periodic Limb Movement Disorder (PLMD), Restless Leg Syndrome (RLS), Sleep-Disordered Breathing (SDB), Obstructive Sleep Apnea (OSA), Central Sleep Apnea (CSA), other types of apneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, insomnia, and chest wall disorders.

Obstructive Sleep Apnea (OSA), a form of Sleep Disordered Breathing (SDB), is characterized by events including occlusion or obstruction of the upper air passage during sleep resulting from a combination of an abnormally small upper airway and the normal loss of muscle tone in the region of the tongue, soft palate and posterior oropharyngeal wall.

Central Sleep Apnea (CSA) is another form of sleep disordered breathing. CSA results when the brain temporarily stops sending signals to the muscles that control breathing. Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

A Respiratory Effort Related Arousal (RERA) event is typically characterized by an increased respiratory effort for ten seconds or longer leading to arousal from sleep and which does not fulfill the criteria for an apnea or hypopnea event. RERAs are defined as a sequence of breaths characterized by increasing respiratory effort leading to an arousal from sleep, but which does not meet criteria for an apnea or hypopnea. These events must fulfil both of the following criteria: (1) a pattern of progressively more negative esophageal pressure, terminated by a sudden change in pressure to a less negative level and an arousal, and (2) the event lasts ten seconds or longer. In some implementations, a Nasal Cannula/Pressure Transducer System is adequate and reliable in the detection of RERAs. A RERA detector may be based on a real flow signal derived from a respiratory therapy device. For example, a flow limitation measure may be determined based on a flow signal. A measure of arousal may then be derived as a function of the flow limitation measure and a measure of sudden increase in ventilation. One such method is described in WO 2008/138040 and U.S. Patent No. 9,358,353, assigned to ResMed Ltd.

Cheyne-Stokes Respiration (CSR) is a further form of SDB. CSR is a disorder of a patient's respiratory controller in which there are rhythmic alternating periods of waxing and waning ventilation known as CSR cycles. CSR is characterized by repetitive de-oxygenation and re-oxygenation of the arterial blood. Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness. Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung. Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

Many of these disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that can occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

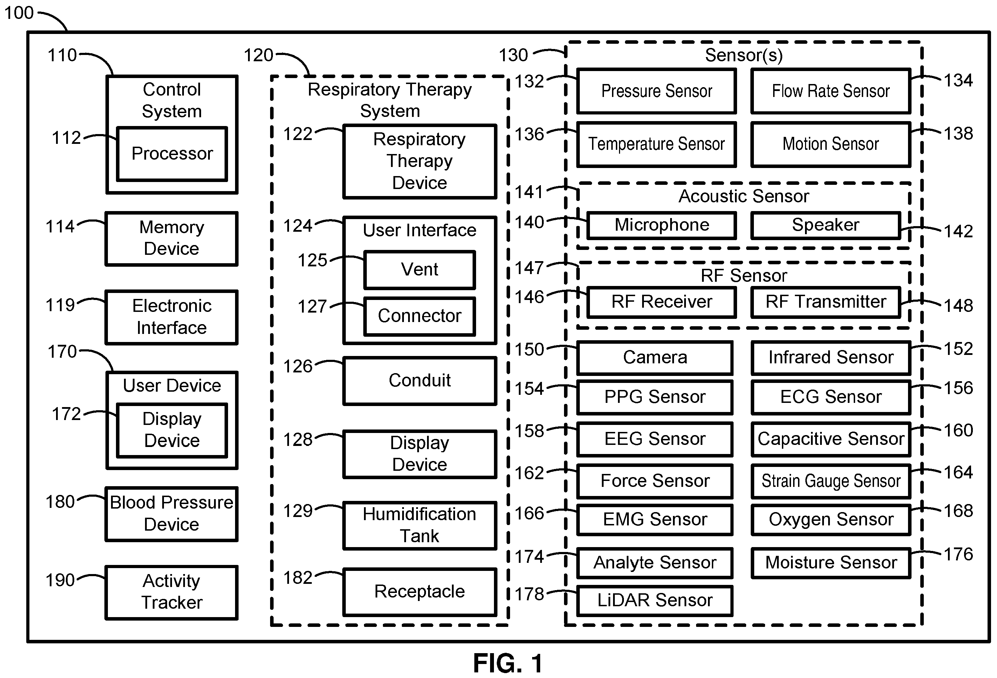

Referring to FIG. 1, a system 100, according to some implementations of the present disclosure, is illustrated. The system 100 includes a control system 110, a memory device 114, an electronic interface 119, one or more sensors 130, and optionally one or more user devices 170. In some implementations, the system 100 further includes a respiratory therapy system 120 (that includes a respiratory therapy device 122), a blood pressure device 180, an activity tracker 190, or any combination thereof. The system 100 can be used to modify the pressure settings of the respiratory therapy system.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 (or any other control system) or a portion of the control system 110 such as the processor 112 (or any other processor(s) or portion(s) of any other control system), can be used to carry out one or more steps of any of the methods described and/or claimed herein. The control system 110 can be coupled to and/or positioned within, for example, a housing of the user device 170, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions thereon that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of a respiratory therapy device 122 of the respiratory therapy system 120, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a family medical history (such as a family history of insomnia or sleep apnea), an employment status of the user, an educational status of the user, a socioeconomic status of the user, or any combination thereof. The medical information can include, for example, information indicative of one or more medical conditions associated with the user, medication usage by the user, or both. The medical information data can further include a fall risk assessment associated with the user (e.g., a fall risk score using the Morse fall scale), a multiple sleep latency test (MSLT) result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or acoustic data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, an IR communication protocol, over a cellular network, over any other optical communication protocol, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 optionally includes a respiratory therapy system 120 (also referred to as a respiratory pressure therapy system). The respiratory therapy system 120 can include a respiratory therapy device 122 (also referred to as a respiratory pressure device), a user interface 124 (also referred to as a mask or a patient interface), a conduit 126 (also referred to as a tube or an air circuit), a display device 128, a humidification tank 129, a receptacle 182, or any combination thereof. In some implementations, the control system 110, the memory device 114, the display device 128, one or more of the sensors 130, the humidification tank 129, and the receptacle 182 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea), other respiratory disorders such as COPD, or other disorders leading to respiratory insufficiency, that may manifest either during sleep or wakefulness.

The respiratory therapy device 122 is generally used to generate pressurized air that is delivered to a user (e.g., using one or more motors (such as a blower motor) that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 cm $H_2O$, at least about 10 cm $H_2O$, at least about 20 cm $H_2O$, between about 6 cm $H_2O$ and about 10 cm $H_2O$, between about 7 cm $H_2O$ and about 12 cm $H_2O$, etc. The respiratory therapy device 122 can also deliver pressurized air at a predetermined flow rate between, for example, about -20 L/min and about 150 L/min, while maintaining a positive pressure (relative to the ambient pressure). In some implementations, the control system 110, the memory device 114, the electronic interface 119, or any combination thereof can be coupled to and/or positioned within a housing of the respiratory therapy device 122.

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm $H_2O$ relative to ambient pressure. For other forms of therapy, such as the delivery of oxygen, the user interface may not include a seal sufficient to facilitate delivery to the airways of a supply of gas at a positive pressure of about 10 cm $H_2O$.

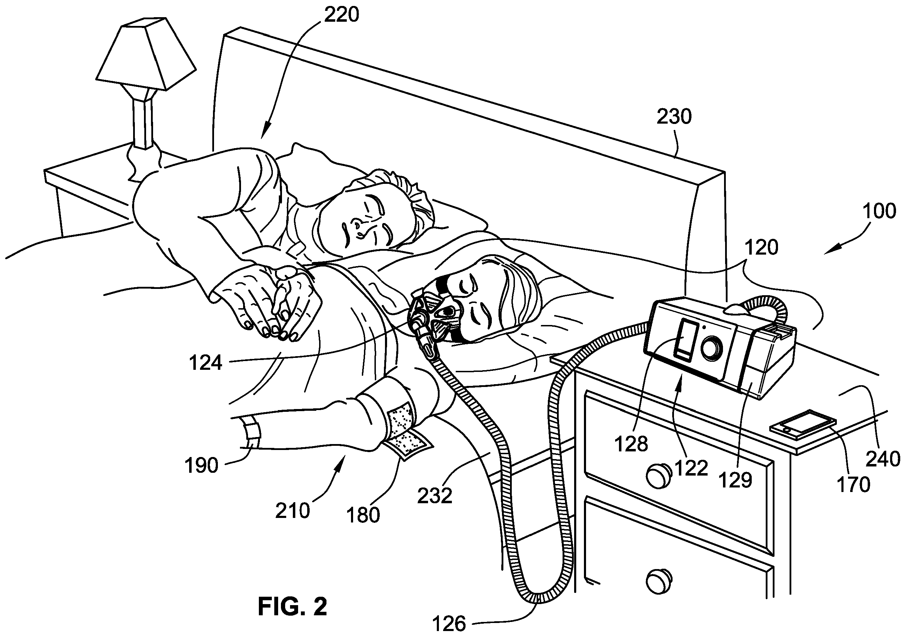

In some implementations, the user interface 124 is or includes a facial mask that covers the nose and mouth of the user (as shown, for example, in FIG. 2). Alternatively, the user interface 124 is or includes a nasal mask that provides air to the nose of the user or a nasal pillow mask that delivers air directly to the nostrils of the user. The user interface 124 can include a strap assembly that has a plurality of straps (e.g., including hook and loop fasteners) for positioning and/or stabilizing the user interface 124 on a portion of the user interface 124 on a desired location of the user (e.g., the face), and a conformal cushion (e.g., silicone, plastic, foam, etc.) that aids in providing an air-tight seal between the user interface 124 and the user. In some implementations, the user interface 124 may include a connector 127 and one or more vents 125. The one or more vents 125 can be used to permit the escape of carbon dioxide and other gases exhaled by the user. In other implementations, the user interface 124 includes a mouthpiece (e.g., a night guard mouthpiece molded to conform to the user's teeth, a mandibular repositioning device, etc.). In some implementations, the connector 127 is distinct from, but couplable to, the user interface 124 (and/or conduit 126). The connector 127 is configured to connect and fluidly couple the user interface 124 to the conduit 126.

The conduit 126 allows the flow of air between two components of a respiratory therapy system 120, such as the respiratory therapy device 122 and the user interface 124. In some implementations, there can be separate limbs of the conduit for inhalation and exhalation. In other implementations, a single limb conduit is used for both inhalation and exhalation. Generally, the respiratory therapy system 120 forms an air pathway that extends between a motor of the respiratory therapy device 122 and the user and/or the user's airway. Thus, the air pathway generally includes at least a motor of the respiratory therapy device 122, the user interface 124, and the conduit 126.

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score or a therapy score (such as a myAir™ score, such as described in WO 2016/061629 and US2017/0311879), the current date/time, personal information for the user, a questionnaire for the user, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122 and includes a reservoir of water that can be used to humidify the pressurized air delivered from the respiratory therapy device 122. The respiratory therapy device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself. In other implementations, the respiratory therapy device 122 or the conduit 126 can include a waterless humidifier. The waterless humidifier can incorporate sensors that interface with other sensor positioned elsewhere in the system 100.

In some implementations, the system 100 can be used to deliver at least a portion of a substance from the receptacle 182 to the air pathway of the user based at least in part on the physiological data, the sleep-related parameters, other data or information, or any combination thereof. Generally, modifying the delivery of the portion of the substance into the air pathway can include (i) initiating the delivery of the substance into the air pathway, (ii) ending the delivery of the portion of the substance into the air pathway, (iii) modifying an amount of the substance delivered into the air pathway, (iv) modifying a temporal characteristic of the delivery of the portion of the substance into the air pathway, (v) modifying a quantitative characteristic of the delivery of the portion of the substance into the air pathway, (vi) modifying any parameter associated with the delivery of the substance into the air pathway, or (vii) a combination of (i)-(vi).

Modifying the temporal characteristic of the delivery of the portion of the substance into the air pathway can include changing the rate at which the substance is delivered, starting and/or finishing at different times, continuing for different time periods, changing the time distribution or characteristics of the delivery, changing the amount distribution independently of the time distribution, etc. The independent time and amount variation ensures that, apart from varying the frequency of the release of the substance, one can vary the amount of substance released each time. In this manner, a number of different combination of release frequencies and release amounts (e.g., higher frequency but lower release amount, higher frequency and higher amount, lower frequency and higher amount, lower frequency and lower amount, etc.) can be achieved. Other modifications to the delivery of the portion of the substance into the air pathway can also be utilized.

The respiratory therapy system 120 can be used, for example, as a ventilator or a positive airway pressure (PAP) system, such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based at least in part on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 (e.g., a full facial mask) can be worn by the user 210 during a sleep session. The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can include the display device 128, which can allow the user to interact with the respiratory therapy device 122. The respiratory therapy device 122 can also include the humidification tank 129, which stores the water used to humidify the pressurized air. The respiratory therapy device 122 can be positioned on a nightstand 240 that is directly adjacent to the bed 230 as shown in FIG. 2, or more generally, on any surface or structure that is generally adjacent to the bed 230 and/or the user 210. The user can also wear the blood pressure device 180 and the activity tracker 190 while lying on the mattress 232 in the bed 230.

Referring back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, an RF transmitter 148, a camera 150, an infrared (IR) sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a light detection and ranging (LiDAR) sensor 178, or any combination thereof. Generally, each of the one or sensors 130 are configured to output sensor data that is received and stored in the memory device 114 or one or more other memory devices. The sensors 130 can also include, an electrooculography (EOG) sensor, a peripheral oxygen saturation ($SpO_2$) sensor, a galvanic skin response (GSR) sensor, a carbon dioxide ($CO_2$) sensor, or any combination thereof.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the IR sensor 152, the PPG sensor 154, the ECG sensor 156, the EEG sensor 158, the capacitive sensor 160, the force sensor 162, the strain gauge sensor 164, the EMG sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example physiological data, acoustic data, or both, that is associated with a user of the respiratory therapy system 120 (such as the user 210 of FIG. 2), the respiratory therapy system 120, both the user and the respiratory therapy system 120, or other entities, objects, activities, etc. Physiological data generated by one or more of the sensors 130 can be used by the control system 110 to determine a sleep-wake signal associated with the user during the sleep session and one or more sleep-related parameters. The sleep-wake signal can be indicative of one or more sleep stages (sometimes referred to as sleep states), including sleep, wakefulness, relaxed wakefulness, micro-awakenings, or distinct sleep stages such as a rapid eye movement (REM) stage (which can include both a typical REM stage and an atypical REM stage), a first non-REM stage (often referred to as "N1"), a second non-REM stage (often referred to as "N2"), a third non-REM stage (often referred to as "N3"), or any combination thereof. Methods for determining sleep stages from physiological data generated by one or more of the sensors, such as sensors 130, are described in, for example, WO 2014/047310, US 10,492,720, US 10,660,563, US 2020/0337634, WO 2017/132726, WO 2019/122413, US 2021/0150873, WO 2019/122414, US2020/0383580.

The sleep-wake signal can also be timestamped to indicate a time that the user enters the bed, a time that the user exits the bed, a time that the user attempts to fall asleep, etc. The sleep-wake signal can be measured one or more of the sensors 130 during the sleep session at a predetermined sampling rate, such as, for example, one sample per second, one sample per 30 seconds, one sample per minute, etc. Examples of the one or more sleep-related parameters that can be determined for the user during the sleep session based at least in part on the sleep-wake signal include a total time in bed, a total sleep time, a total wake time, a sleep onset latency, a wake-after-sleep-onset parameter, a sleep efficiency, a fragmentation index, an amount of time to fall asleep, a consistency of breathing rate, a fall asleep time, a wake time, a rate of sleep disturbances, a number of movements, or any combination thereof.

Physiological data and/or acoustic data generated by the one or more sensors 130 can also be used to determine a respiration signal associated with the user during a sleep session. the respiration signal is generally indicative of respiration or breathing of the user during the sleep session. The respiration signal can be indicative of, for example, a respiration rate, a respiration rate variability, an inspiration amplitude, an expiration amplitude, an inspiration-expiration amplitude ratio, an inspiration-expiration duration ratio, a number of events per hour, a pattern of events, pressure settings of the respiratory therapy device 122, or any combination thereof. The event(s) can include snoring, apneas, central apneas, obstructive apneas, mixed apneas, hypopneas, RERAs, a flow limitation (e.g., an event that results in the absence of the increase in flow despite an elevation in negative intrathoracic pressure indicating increased effort), a mask leak (e.g., from the user interface 124), a restless leg, a sleeping disorder, choking, an increased heart rate, a heart rate variation, labored breathing, an asthma attack, an epileptic episode, a seizure, a fever, a cough, a sneeze, a snore, a gasp, the presence of an illness such as the common cold or the flu, an elevated stress level, etc. Events can be detected by any means known in the art such as described in, for example, US 5,245,995, US 6,502,572, WO 2018/050913, WO 2020/104465.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122. The pressure sensor 132 can be, for example, a capacitive sensor, an electromagnetic sensor, an inductive sensor, a resistive sensor, a piezoelectric sensor, a strain-gauge sensor, an optical sensor, a potentiometric sensor, or any combination thereof. In one example, the pressure sensor 132 can be used to determine a blood pressure of the user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user, a skin temperature of the user, a temperature of the air flowing from the respiratory therapy device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user during the sleep session, and/or detect movement of any of the components of the respiratory therapy system 120, such as the respiratory therapy device 122, the user interface 124, or the conduit 126. The motion sensor 138 can include one or more inertial sensors, such as accelerometers, gyroscopes, and magnetometers. The motion sensor 138 can be used to detect motion or acceleration associated with arterial pulses, such as pulses in or around the face of the user and proximal to the user interface 124, and configured to detect features of the pulse shape, speed, amplitude, or volume. In some implementations, the motion sensor 138 alternatively or additionally generates one or more signals representing bodily movement of the user, from which may be obtained a signal representing a sleep state of the user; for example, via a respiratory movement of the user.

The microphone 140 outputs acoustic data that can be stored in the memory device 114 and/or analyzed by the processor 112 of the control system 110. The acoustic data generated by the microphone 140 is reproducible as one or more sound(s) during a sleep session (e.g., sounds from the user) to determine (e.g., using the control system 110) one or more sleep-related parameters, as described in further detail herein. The acoustic data from the microphone 140 can also be used to identify (e.g., using the control system 110) an event experienced by the user during the sleep session, as described in further detail herein. In other implementations, the acoustic data from the microphone 140 is representative of noise associated with the respiratory therapy system 120. In some implementations, the acoustic data from the microphone 140 can be analyzed to detect the presence of liquid in the respiratory therapy system 120, in particular in the user interface 124 and/or the conduit 126, as explained in further detail herein. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones. The microphone 140 can be coupled to or integrated in the respiratory therapy system 120 (or the system 100) generally in any configuration. For example, the microphone 140 can be disposed inside the respiratory therapy device 122, the user interface 124, the conduit 126, or other components. The microphone 140 can also be positioned adjacent to or coupled to the outside of the respiratory therapy device 122, the outside of the user interface 124, the outside of the conduit 126, or outside of any other components. The microphone 140 could also be a component of the user device 170 (e.g., the microphone 140 is a microphone of a smart phone). The microphone 140 can be integrated into the user interface 124, the conduit 126, the respiratory therapy device 122, or any combination thereof. In general, the microphone 140 can be located at any point within or adjacent to the air pathway of the respiratory therapy system 120, which includes at least the motor of the respiratory therapy device 122, the user interface 124, and the conduit 126. Thus, the air pathway can also be referred to as the acoustic pathway.

The speaker 142 outputs sound waves that are typically audible to the user. In one or more implementations, the sound waves can be audible to a user of the system 100 or inaudible to the user of the system (e.g., ultrasonic sound waves). The speaker 142 can be used, for example, as an alarm clock or to play an alert or message to the user (e.g., in response to an event). In some implementations, the speaker 142 can be used to communicate the acoustic data generated by the microphone 140 to the user. The speaker 142 can be coupled to or integrated in the respiratory therapy device 122, the user interface 124, the conduit 126, or the user device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and/or frequency, and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user or a bed partner of the user (such as bed partner 220 in FIG. 2). Based at least in part on the data from the microphone 140 and/or the speaker 142, the control system 110 can determine a location of the user and/or one or more of the sleep-related parameters described in herein, such as, for example, a respiration signal, a respiration rate, an inspiration amplitude, an expiration amplitude, an inspiration-expiration ratio, a number of events per hour, a pattern of events, a sleep stage, pressure settings of the respiratory therapy device 122, a mouth leak status, or any combination thereof. In this context, a SONAR sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO 2018/050913 and WO 2020/104465 mentioned above. In some implementations, the speaker 142 is a bone conduction speaker. In some implementations, the one or more sensors 130 include (i) a first microphone that is the same or similar to the microphone 140, and is integrated into the acoustic sensor 141 and (ii) a second microphone that is the same as or similar to the microphone 140, but is separate and distinct from the first microphone that is integrated into the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147 (e.g., a RADAR sensor). In some such implementations, the RF sensor 147 includes a control circuit. The specific format of the RF communication could be WiFi, Bluetooth, etc.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based at least in part on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user enters the user's bed (such as bed 230 in FIG. 2), and to determine a time when the user exits the bed 230. The camera 150 can also be used to track eye movements, pupil dilation (if one or both of the user's eyes are open), blink rate, or any changes during REM sleep. The camera 150 can also be used to track the position of the user, which can impact the duration and/or severity of apneic episodes in users with positional obstructive sleep apnea.

The IR sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user and/or movement of the user. The IR sensor 152 can also be used in conjunction with the camera 150 when measuring the presence, location, and/or movement of the user. The IR sensor 152 can detect infrared light having a wavelength between about 700 nm and about 1 mm, for example, while the camera 150 can detect visible light having a wavelength between about 380 nm and about 740 nm.

The IR sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user and/or movement of the user. The IR sensor 152 can also be used in conjunction with the camera 150 when measuring the presence, location, and/or movement of the user. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user that can be used to determine one or more sleep-related parameters, such as, for example, a heart rate, a heart rate pattern, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user, embedded in clothing and/or fabric that is worn by the user, embedded in and/or coupled to the user interface 124 and/or its associated headgear (e.g., straps, etc.), etc.

The ECG sensor 156 outputs physiological data associated with electrical activity of the heart of the user. In some implementations, the ECG sensor 156 includes one or more electrodes that are positioned on or around a portion of the user during the sleep session. The physiological data from the ECG sensor 156 can be used, for example, to determine one or more of the sleep-related parameters described herein.

The EEG sensor 158 outputs physiological data associated with electrical activity of the brain of the user. In some implementations, the EEG sensor 158 includes one or more electrodes that are positioned on or around the scalp of the user during the sleep session. The physiological data from the EEG sensor 158 can be used, for example, to determine a sleep stage and/or a sleep state of the user at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user. The data output by the analyte sensor 174 can be stored in the memory device 114 and used by the control system 110 to determine the identity and concentration of any analytes in the user's breath. In some implementations, the analyte sensor 174 is positioned near a mouth of the user to detect analytes in breath exhaled from the user's mouth. For example, when the user interface 124 is a facial mask that covers the nose and mouth of the user, the analyte sensor 174 can be positioned within the facial mask to monitor the user mouth breathing. In other implementations, such as when the user interface 124 is a nasal mask or a nasal pillow mask, the analyte sensor 174 can be positioned near the nose of the user to detect analytes in breath exhaled through the user's nose. In still other implementations, the analyte sensor 174 can be positioned near the user's mouth when the user interface 124 is a nasal mask or a nasal pillow mask. In this implementation, the analyte sensor 174 can be used to detect whether any air is inadvertently leaking from the user's mouth. In some implementations, the analyte sensor 174 is a volatile organic compound (VOC) sensor that can be used to detect carbon-based chemicals or compounds, such as carbon dioxide. In some implementations, the analyte sensor 174 can also be used to detect whether the user is breathing through their nose or mouth. For example, if the data output by an analyte sensor 174 positioned near the mouth of the user or within the facial mask (in implementations where the user interface 124 is a facial mask) detects the presence of an analyte, the control system 110 can use this data as an indication that the user is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user's face, near the connection between the conduit 126 and the user interface 124, near the connection between the conduit 126 and the respiratory therapy device 122, etc.). Thus, in some implementations, the moisture sensor 176 can be coupled to or integrated into the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user, for example the air inside the user's bedroom. The moisture sensor 176 can also be used to track the user's biometric response to environmental changes.

One or more LiDAR sensors 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 178 can measure and map an area extending 5 meters or more away from the sensor. The LiDAR data can be fused with point cloud data estimated by an electromagnetic RADAR sensor, for example. The LiDAR sensor 178 may also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the respiratory therapy device 122, the user interface 124, the conduit 126, the humidification tank 129, the control system 110, the user device 170, or any combination thereof. For example, the acoustic sensor 141 and/or the RF sensor 147 can be integrated in and/or coupled to the user device 170. In such implementations, the user device 170 can be considered a secondary device that generates additional or secondary data for use by the system 100 (e.g., the control system 110) according to some aspects of the present disclosure. In some implementations, the pressure sensor 132 and/or the flow rate sensor 134 are integrated into and/or coupled to the respiratory therapy device 122. In some implementations, at least one of the one or more sensors 130 is not coupled to the respiratory therapy device 122, the control system 110, or the user device 170, and is positioned generally adjacent to the user during the sleep session (e.g., positioned on or in contact with a portion of the user, worn by the user, coupled to or positioned on the nightstand, coupled to the mattress, coupled to the ceiling, etc.). More generally, the one or more sensors 130 can be positioned at any suitable location relative to the user such that the one or more sensors 130 can generate physiological data associated with the user and/or the bed partner 220 during one or more sleep session.

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, an average duration of events, a range of event durations, a ratio between the number of different events, a sleep stage, an apnea-hypopnea index (AHI), or any combination thereof. The one or more events can include snoring, apneas, central apneas, obstructive apneas, mixed apneas, hypopneas, an intentional user interface leak, an unintentional user interface leak, a mouth leak, a cough, a restless leg, a sleeping disorder, choking, an increased heart rate, labored breathing, an asthma attack, an epileptic episode, a seizure, increased blood pressure, hyperventilation, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, or from other types of data.

The user device 170 includes a display device 172. The user device 170 can be, for example, a mobile device such as a smart phone, a tablet, a laptop, a gaming console, a smart watch, or the like. Alternatively, the user device 170 can be an external sensing system, a television (e.g., a smart television) or another smart home device (e.g., a smart speaker(s) such as Google Home®, Google Nest®, Amazon Echo®, Amazon Echo Show®, Alexa®-enabled devices, etc.). In some implementations, the user device 170 is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices 170 can be used by and/or included in the system 100.

The blood pressure device 180 is generally used to aid in generating physiological data for determining one or more blood pressure measurements associated with a user. The blood pressure device 180 can include at least one of the one or more sensors 130 to measure, for example, a systolic blood pressure component and/or a diastolic blood pressure component.

In some implementations, the blood pressure device 180 is a sphygmomanometer including an inflatable cuff that can be worn by a user and a pressure sensor (e.g., the pressure sensor 132 described herein). For example, as shown in the example of FIG. 2, the blood pressure device 180 can be worn on an upper arm of the user. In such implementations where the blood pressure device 180 is a sphygmomanometer, the blood pressure device 180 also includes a pump (e.g., a manually operated bulb) for inflating the cuff. In some implementations, the blood pressure device 180 is coupled to the respiratory therapy device 122 of the respiratory therapy system 120, which in turn delivers pressurized air to inflate the cuff. More generally, the blood pressure device 180 can be communicatively coupled with, and/or physically integrated in (e.g., within a housing), the control system 110, the memory device 114, the respiratory therapy system 120, the user device 170, and/or the activity tracker 190.

The activity tracker 190 is generally used to aid in generating physiological data for determining an activity measurement associated with the user. The activity measurement can include, for example, a number of steps, a distance traveled, a number of steps climbed, a duration of physical activity, a type of physical activity, an intensity of physical activity, time spent standing, a respiration rate, an average respiration rate, a resting respiration rate, a maximum respiration rate, a respiration rate variability, a heart rate, an average heart rate, a resting heart rate, a maximum heart rate, a heart rate variability, a number of calories burned, blood oxygen saturation, electrodermal activity (also known as skin conductance or galvanic skin response), or any combination thereof. The activity tracker 190 includes one or more of the sensors 130 described herein, such as, for example, the motion sensor 138 (e.g., one or more accelerometers and/or gyroscopes), the PPG sensor 154, and/or the ECG sensor 156.

In some implementations, the activity tracker 190 is a wearable device that can be worn by the user, such as a smartwatch, a wristband, a ring, or a patch. For example, referring to FIG. 2, the activity tracker 190 is worn on a wrist of the user. The activity tracker 190 can also be coupled to or integrated a garment or clothing that is worn by the user.

Alternatively, still, the activity tracker 190 can also be coupled to or integrated in (e.g., within the same housing) the user device 170. More generally, the activity tracker 190 can be communicatively coupled with, or physically integrated in (e.g., within a housing), the control system 110, the memory device 114, the respiratory therapy system 120, the user device 170, and/or the blood pressure device 180.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for modifying pressure settings, according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. As a further example, a fourth alternative system includes the control system 110, the memory device 114, the respiratory therapy system 120, at least one of the one or more sensors 130, the user device 170, and the blood pressure device 180 and/or activity tracker 190. Thus, various systems for modifying pressure settings can be formed using any portion or portions of the components shown and described herein and/or in combination with one or more other components.

Referring again to FIG. 2, in some implementations, the control system 110, the memory device 114, any of the one or more sensors 130, or a combination thereof can be located on and/or in any surface and/or structure that is generally adjacent to the bed 230 and/or the user 210. For example, in some implementations, at least one of the one or more sensors 130 can be located at a first position on and/or in one or more components of the respiratory therapy system 120 adjacent to the bed 230 and/or the user 210. The one or more sensors 130 can be coupled to the respiratory therapy system 120, the user interface 124, the conduit 126, the display device 128, the humidification tank 129, or a combination thereof.

Alternatively, or additionally, at least one of the one or more sensors 130 can be located at a second position on and/or in the bed 230 (e.g., the one or more sensors 130 are coupled to and/or integrated in the bed 230). Further, alternatively or additionally, at least one of the one or more sensors 130 can be located at a third position on and/or in the mattress 232 that is adjacent to the bed 230 and/or the user 210 (e.g., the one or more sensors 130 are coupled to and/or integrated in the mattress 232). Alternatively, or additionally, at least one of the one or more sensors 130 can be located at a fourth position on and/or in a pillow that is generally adjacent to the bed 230 and/or the user 210.

Alternatively, or additionally, at least one of the one or more sensors 130 can be located at a fifth position on and/or in the nightstand 240 that is generally adjacent to the bed 230 and/or the user 210. Alternatively, or additionally, at least one of the one or more sensors 130 can be located at a sixth position such that the at least one of the one or more sensors 130 are coupled to and/or positioned on the user 210 (e.g., the one or more sensors 130 are embedded in or coupled to fabric, clothing, and/or a smart device worn by the user 210). More generally, at least one of the one or more sensors 130 can be positioned at any suitable location relative to the user 210 such that the one or more sensors 130 can generate sensor data associated with the user 210.

In some implementations, a primary sensor, such as the microphone 140, is configured to generate acoustic data associated with the user 210 during a sleep session. The acoustic data can be based on, for example, acoustic signals in the conduit 126 of the respiratory therapy system 120. For example, one or more microphones (the same as, or similar to, the microphone 140 of FIG. 1) can be integrated in and/or coupled to (i) a circuit board of the respiratory therapy device 122, (ii) the conduit 126, (iii) a connector between components of the respiratory therapy system 120, (iv) the user interface 124, (v) a headgear (e.g., straps) associated with the user interface, or (vi) a combination thereof. In some implementations, the microphone 140 is in fluid communication with the airflow pathway (e.g., an airflow pathway between the flow generator/motor and the distal end of the conduit). By fluid communication, it is intended to also include configurations wherein the microphone is in acoustic communication with the airflow pathway without being in direct or physical contact with the airflow. For example, in some implementations, the microphone is positioned on a circuit board and in fluid communication, optionally via a duct sealed by a membrane, to the airflow pathway.

In some implementations, one or more secondary sensors may be used in addition to the primary sensor to generate additional data. In some such implementations, the one or more secondary sensors include: a microphone (e.g., the microphone 140 of the system 100), a flow rate sensor (e.g., the flow rate sensor 134 of the system 100), a pressure sensor (e.g., the pressure sensor 132 of the system 100), a temperature sensor (e.g., the temperature sensor 136 of the system 100), a camera (e.g., the camera 150 of the system 100), a vane sensor (VAF), a hot wire sensor (MAF), a cold wire sensor, a laminar flow sensor, an ultrasonic sensor, an inertial sensor, or a combination thereof.

Additionally, or alternatively, one or more microphones (the same as, or similar to, the microphone 140 of FIG. 1) can be integrated in and/or coupled to a co-located smart device, such as the user device 170, a TV, a watch (e.g., a mechanical watch or another smart device worn by the user), a pendant, the mattress 232, the bed 230, beddings positioned on the bed 230, the pillow, a speaker (e.g., the speaker 142 of FIG. 1), a radio, a tablet device, a waterless humidifier, or a combination thereof. A co-located smart device can be any smart device that is within range for detecting sounds emitted by the user, the respiratory therapy system 120, and/or any portion of the system 100. In some implementations, the co-located smart device is a smart device that is in the same room as the user during the sleep session.

Additionally, or alternatively, in some implementations, one or more microphones (the same as, or similar to, the microphone 140 of FIG. 1) can be remote from the system 100 (FIG. 1) and/or the user 210 (FIG. 2), so long as there is an air passage allowing acoustic signals to travel to the one or more microphones. For example, the one or more microphones can be in a different room from the room containing the system 100.

As used herein, a sleep session can be defined in a number of ways based at least in part on, for example, an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the user device 170 (FIG. 1) to manually initiate or terminate the sleep session.

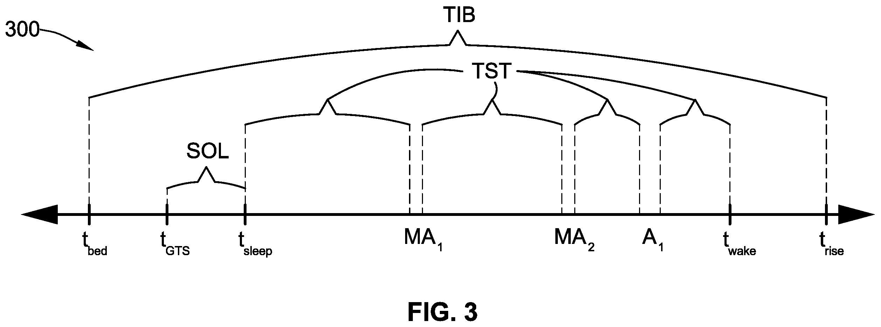

Referring to FIG. 3, an exemplary timeline 300 for a sleep session is illustrated. The timeline 300 includes an enter bed time ($t_{bed}$), a go-to-sleep time ($t_{GTS}$), an initial sleep time ($t_{sleep}$), a first micro-awakening $MA_1$, a second micro-awakening $MA_2$, an awakening A, a wake-up time ($t_{wake}$), and a rising time ($t_{rise}$).

The enter bed time $t_{bed}$ is associated with the time that the user initially enters the bed (e.g., bed 230 in FIG. 2) prior to falling asleep (e.g., when the user lies down or sits in the bed). The enter bed time $t_{bed}$ can be identified based at least in part on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time $t_{bed}$ is described herein in reference to a bed, more generally, the enter time $t_{bed}$ can refer to the time the user initially enters any location for sleeping (e.g., a couch, a chair, a sleeping bag, etc.).

The go-to-sleep time (GTS) is associated with the time that the user initially attempts to fall asleep after entering the bed ($t_{bed}$). For example, after entering the bed, the user may engage in one or more activities to wind down prior to trying to sleep (e.g., reading, watching TV, listening to music, using the user device 170, etc.). The initial sleep time ($t_{sleep}$) is the time that the user initially falls asleep. For example, the initial sleep time ($t_{sleep}$) can be the time that the user initially enters the first non-REM sleep stage.

The wake-up time $t_{wake}$ is the time associated with the time when the user wakes up without going back to sleep (e.g., as opposed to the user waking up in the middle of the night and going back to sleep). The user may experience one of more unconscious microawakenings (e.g., microawakenings $MA_1$ and $MA_2$) having a short duration (e.g., 5 seconds, 10 seconds, 30 seconds, 1 minute, etc.) after initially falling asleep. In contrast to the wake-up time $t_{wake}$, the user goes back to sleep after each of the microawakenings $MA_1$ and $MA_2$. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time $t_{wake}$ can be defined, for example, based at least in part on a wake threshold duration (e.g., the user is awake for at least 15 minutes, at least 20 minutes, at least 30 minutes, at least 1 hour, etc.).

Similarly, the rising time $t_{rise}$ is associated with the time when the user exits the bed and stays out of the bed with the intent to end the sleep session (e.g., as opposed to the user getting up during the night to go to the bathroom, to attend to children or pets, sleep walking, etc.). In other words, the rising time $t_{rise}$ is the time when the user last leaves the bed without returning to the bed until a next sleep session (e.g., the following evening). Thus, the rising time $t_{rise}$ can be defined, for example, based at least in part on a rise threshold duration (e.g., the user has left the bed for at least 15 minutes, at least 20 minutes, at least 30 minutes, at least 1 hour, etc.). The enter bed time $t_{bed}$ time for a second, subsequent sleep session can also be defined based at least in part on a rise threshold duration (e.g., the user has left the bed for at least 4 hours, at least 6 hours, at least 8 hours, at least 12 hours, etc.).

As described above, the user may wake up and get out of bed one more times during the night between the initial $t_{bed}$ and the final $t_{rise}$. In some implementations, the final wake-up time $t_{wake}$ and/or the final rising time $t_{rise}$ that are identified or determined based at least in part on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up ($t_{wake}$) or raising up ($t_{rise}$), and the user either going to bed ($t_{bed}$), going to sleep ($t_{GTS}$) or falling asleep ($t_{sleep}$) of between about 12 and about 18 hours can be used. For users that spend longer periods of time in bed, shorter threshold periods may be used (e.g., between about 8 hours and about 14 hours). The threshold period may be initially selected and/or later adjusted based at least in part on the system monitoring the user's sleep behavior.

The total time in bed (TIB) is the duration of time between the time enter bed time $t_{bed}$ and the rising time $t_{rise}$. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 300 of FIG. 3, the total sleep time (TST) spans between the initial sleep time $t_{sleep}$ and the wake-up time $t_{wake}$, but excludes the duration of the first micro-awakening $MA_1$, the second micro-awakening $MA_2$, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time ($t_{bed}$) and ending at the rising time ($t_{rise}$), i.e., the sleep session is defined as the total time in bed (TIB). In some implementations, a sleep session is defined as starting at the initial sleep time ($t_{sleep}$) and ending at the wake-up time ($t_{wake}$). In some implementations, the sleep session is defined as the total sleep time (TST). In some implementations, a sleep session is defined as starting at the go-to-sleep time ($t_{GTS}$) and ending at the wake-up time ($t_{wake}$). In some implementations, a sleep session is defined as starting at the go-to-sleep time ($t_{GTS}$) and ending at the rising time ($t_{rise}$). In some implementations, a sleep session is defined as starting at the enter bed time ($t_{bed}$) and ending at the wake-up time ($t_{wake}$). In some implementations, a sleep session is defined as starting at the initial sleep time ($t_{sleep}$) and ending at the rising time ($t_{rise}$).

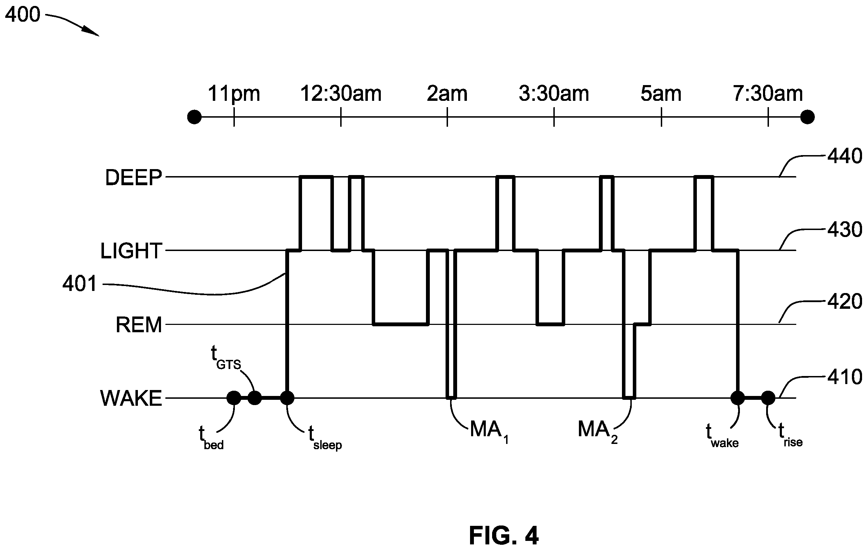

Referring to FIG. 4, an exemplary hypnogram 400 corresponding to the timeline 300 (FIG. 3), according to some implementations, is illustrated. As shown, the hypnogram 400 includes a sleep-wake signal 401, a wakefulness stage axis 410, a REM stage axis 420, a light sleep stage axis 430, and a deep sleep stage axis 440. The intersection between the sleep-wake signal 401 and one of the axes 410-440 is indicative of the sleep stage at any given time during the sleep session.

The sleep-wake signal 401 can be generated based at least in part on physiological data associated with the user (e.g., generated by one or more of the sensors 130 described herein). The sleep-wake signal can be indicative of one or more sleep stages, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 400 is shown in FIG. 4 as including the light sleep stage axis 430 and the deep sleep stage axis 440, in some implementations, the hypnogram 400 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration amplitude ratio, an inspiration-expiration duration ratio, a number of events per hour, a pattern of events, or any combination thereof. Information describing the sleep-wake signal can be stored in the memory device 114.

The hypnogram 400 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time ($t_{GTS}$) and the initial sleep time ($t_{sleep}$). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings $MA_1$ and $MA_2$ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based at least in part on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening $MA_1$ and micro-awakening $MA_2$ shown in FIG. 4), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time ($t_{bed}$), the go-to-sleep time ($t_{GTS}$), the initial sleep time ($t_{sleep}$), one or more first micro-awakenings (e.g., $MA_1$ and $MA_2$), the wake-up time ($t_{wake}$), the rising time ($t_{rise}$), or any combination thereof based at least in part on the sleep-wake signal of a hypnogram.

In other implementations, one or more of the sensors 130 can be used to determine or identify the enter bed time ($t_{bed}$), the go-to-sleep time ($t_{GTS}$), the initial sleep time ($t_{sleep}$), one or more first micro-awakenings (e.g., $MA_1$ and $MA_2$), the wake-up time ($t_{wake}$), the rising time ($t_{rise}$), or any combination thereof, which in turn define the sleep session. For example, the enter bed time $t_{bed}$ can be determined based at least in part on, for example, data generated by the motion sensor 138, the microphone 140, the camera 150, or any combination thereof. The go-to-sleep time can be determined based at least in part on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory therapy device 122, data indicative of the user donning the user interface 124, etc.), or any combination thereof.

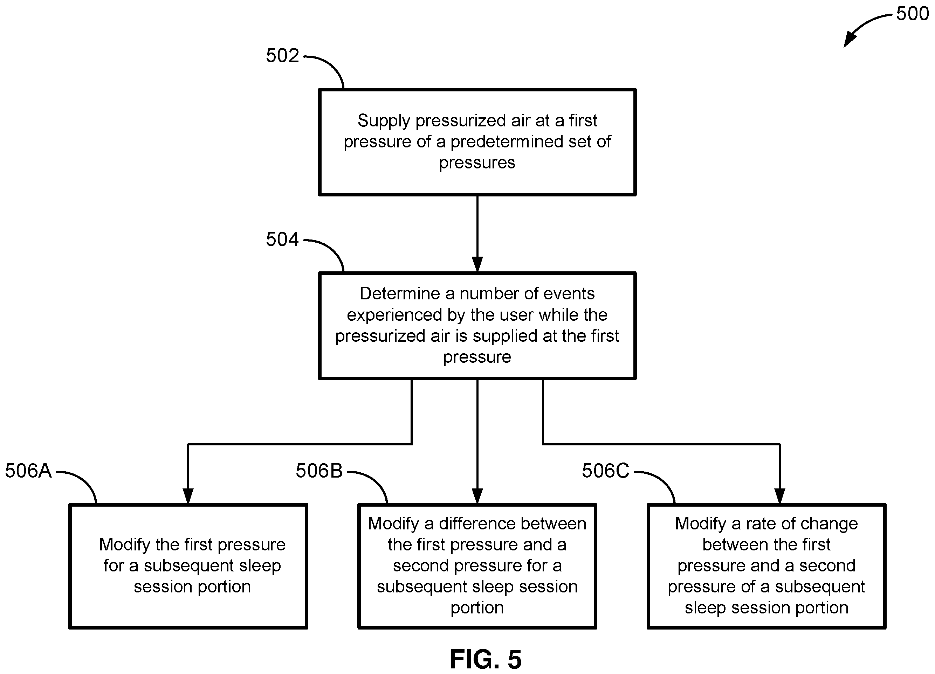

Referring to FIG. 5, a method 500 for modifying pressure settings of a respiratory therapy system (such as respiratory therapy system 120) is illustrated. In some implementations, the respiratory therapy system includes a respiratory therapy device configured to supply pressurized air (such as respiratory therapy device 122), and a user interface (such as user interface 124) coupled to the respiratory therapy device via a conduit (such as conduit 126). The user interface is configured to engage with the user, and aids in directing the pressurized air to the user's airway. Generally, a control system having one or more processors (such as control system 110 of system 100) is configured to carry out the steps of method 500. The control system can be coupled to a memory device (such as memory device 114 of system 100) that stores machine-readable instructions. The machine-readable instructions can be executed by at least one of the one or more processors of the control system to carry out the steps of method 500. The memory device can also store any type of data utilized in the steps of method 500. Generally, method 500 can be implemented using a system (such as system 100) that includes the respiratory therapy system, the control system, and the memory device. Method 500 can also be implemented using a computer program product (such as a non-transitory computer readable medium) comprising instructions that when executed by a computer, cause the computer to carry out the steps of method 500.

Step 502 of method 500 includes supplying pressurized air to a user during a current sleep session. The pressurized air is supplied at a first pressure of a predetermined set or range of pressures. Generally, when a user (such as user 210) begins a sleep session and dons a user interface (such as user interface 124), the respiratory therapy system will begin to supply pressurized air to the airway of the user. In some implementations, the respiratory therapy system may steadily increase the pressure of the pressurized air supplied by the respiratory therapy system until the system determines that the user is asleep. This can be referred to as a "ramp," a "ramp increase," or a "ramp portion" of the sleep session. Once the user is determined to be asleep, the pressurized air may then be supplied at a prescribed therapy pressure, or within a prescribed range of therapy pressures. In other implementations, the pressure of the pressurized air is held constant until the system determines that the user is asleep and/or until a threshold duration of time has passed, e.g., 30 minutes, since the user donned the user interface, and the "ramp" ("ramp increase" or a "ramp portion" of the sleep session) begins once the user is asleep and/or the threshold duration of time has passed. In still other implementations, no pressurized air is supplied to the user until it is determined that the user is asleep

Once it is determined that the user is asleep, the respiratory therapy system can supply pressurized air at a first pressure to the user. Generally, the pressure of the pressurized air that is supplied to the user once the user is asleep is within some predetermined range of pressures (e.g., the range of physician-prescribed therapy pressures). The first pressure is generally at the lower end of this predetermined range of pressures (e.g., the lowermost pressure). During the course of the sleep session, the respiratory therapy system can modify the pressure of the pressurized air in a variety of different manners. For example, the respiratory therapy system can increase the pressure of the pressurized air in a step-wise fashion, where each step (e.g., each increase in pressure) occurs after some predetermined time period.

In other implementations, the respiratory therapy system can increase the pressure of the pressurized air based on the detection of certain events, such as, for example, snoring, central apneas (e.g., a cessation of breathing due to the brain temporarily not sending signals to the muscles controlling breathing), obstructive apneas (e.g., a cessation of breathing due to an obstruction or occlusion of the upper air passage), mixed apneas (e.g., combinations of central apneas and obstructive apneas), hypopneas, choking, etc. The respiratory therapy system may also increase the pressure of the pressurized air based on a body position of the user (e.g., a body position in which the user is more prone to events and thus suffers from, for example, positional SDB such as position OSA) and/or a sleep stage of the user (e.g., the user may be more prone to events when in certain sleep stages, and thus may suffer from, for example, REM-dominant SDB such as REM-dominant OSA). In still further implementations, the respiratory therapy system can decrease the pressure of the pressurized air based on an absence of certain events, the body position of the user, the sleep stage of the user, or any combination thereof.

In some implementations, the predetermined range of pressures is prescribed by a healthcare provider, such as the user's doctor. The prescription can include a variety of different information related to the range of pressures, including a minimum pressure, a maximum pressure, a minimum pressure increase between different steps in the range, a maximum pressure increase between different steps in the range, a minimum amount of time to be spent at a given pressure, a maximum amount of time to be spent at a given pressure, etc.

At step 504, the number of events experienced by the user during a first portion of the current sleep session is determined. Generally, a "portion" of the sleep session can be defined in different ways. In some cases, a portion of the sleep session is defined as the duration between (i) the time when the first pressure of the predetermined range of pressures is applied, and (ii) the time when the first pressure of the predetermined range of pressures is again applied. For example, the pressure may be increased from the first pressure to one or more other pressures in the predetermined range of pressures in response to the user experiencing events during the sleep session (and/or other based on one or more other factors). If no events have occurred after a certain time, the pressure may be lowered back to the first pressure. The time between the pressure initially being at the first pressure and subsequently returning to the first pressure can be defined as a portion of the sleep session. In other cases, a portion of the sleep session may be defined as a fixed amount of time, e.g. X number of minutes or hours. In other cases, a portion of the sleep session may be defined as a fixed number of events occurring. The beginning of a given portion of a sleep session can be when the user initially falls asleep during the sleep session (e.g., when the portion is the very first portion of the sleep session). The beginning of a given portion of a sleep session can also be when pressure of the pressurized air is set to the first pressure in the predetermined range of pressures (which could occur in any portion of the sleep session, whether it is the very first sleep session or not).

With respect to step 504, the first portion of the current sleep session may be the very first portion in the sleep session when the first pressure of the predetermined range of pressures is applied after the user has initially fallen asleep. However, the first portion of the current sleep session could also be any given portion of the sleep session when the first pressure is again applied. During this portion of the sleep session, the user may experience a variety of different events, as described above. The number of events that that the user experiences during this first portion can be determined. Additionally or alternatively, the types of the events and/or the severity of the events (e.g., based on the magnitude of breathing-related flow rate reduction and/or the length of time that breathing-related flow was reduced) experienced during the first portion of the current sleep session are determined. The severity of an event in some implementations is based on the magnitude of breathing-related flow rate reduction during the event, and/or the length of time that breathing-related flow was reduced during the event.

In some implementations, the number, type, and/or severity of events experienced by the user during the current sleep session can be determined based on pressure data associated with the pressure of the pressurized air, flow rate data associated with the flow rate of the pressurized air, or both. This pressure data and flow rate data can be generated by any suitable sensors, including a pressure sensor (such as pressure sensor 132) and/or a flow rate sensor (such as flow rate sensor 134).

The goal of using the predetermined range of pressures for the pressurized air supplied to the user is to gradually increase the pressure of the pressurized air to a level that (i) prevents the user from experiencing events such as apneas, choking, snoring, etc., or (ii) prevents the number of events from satisfying (e.g., exceeding) a predetermined threshold. The predetermined range of pressures is generally designed so that the user's residual AHI (and/or one or more other measures of events) during the sleep session remains below a maximum AHI, (e.g., so that the user does not, over the course of the sleep session (which can comprise or consist of a duration of the sleep session during which the user is receiving respiratory therapy), experience an undesirable average number of events). However, the user can still experience more events than is desirable when the pressure of the pressurized air supplied to the user is at the lower end of the predetermined range of pressures, including the first pressure at the lowermost end of the predetermined range of pressures.

Thus, if the number, type, and/or severity of events that the user experiences during the first portion of the current sleep session satisfy a predetermined threshold, different actions can be taken to modify the pressure settings of the respiratory therapy system in one or more subsequent portions of the current sleep session, and/or one or portions of a subsequent sleep session. The modifications are configured to aid in reducing the user's AHI (and/or one or more other measures of events) during the subsequent sleep session portion of. Steps 506A-506C of method 500 relate to various different modifications that can be made to the pressure settings of the respiratory therapy system during the subsequent sleep session portion.

In some implementations, the portion of the sleep session that is being modified includes any portion of a subsequent sleep session that follows the current sleep session. For example, the portion of the current sleep session could be the first (or later) portion of the current sleep session, and the subsequent portion of the sleep session that is being modified could be the first (or later) portion of the next sleep session (or a later sleep session). Thus, method 500 can be a method for adjusting pressure settings of the user's next sleep session.

In other implementations, the portion of the sleep session that is being modified includes a subsequent portion of the same sleep session (e.g., it is the second portion of the current sleep session that follows the first portion of the current sleep session). For example, the portion of the current sleep session where the events are being monitored could be the first portion of the current sleep session (which could be the very initial portion during which the first pressure is applied, or any portion during which the first pressure is applied), and the subsequent portion of the sleep session that is being modified could be the second portion of the current sleep session, and/or later portions of the current sleep session.

As noted above, the pressure of the pressurized air can be at the first pressure at the beginning of a sleep session portion, and can return to the first pressure within the predetermined range of pressures if no events have occurred for a given time period, thus beginning a new portion of the current sleep session. By remembering what occurred previously during a prior portion of the current sleep session, method 500 can be an on-the-fly method for adjusting the pressure settings during the current sleep session, which avoids a pattern where the therapy is reduced to a pressure at which the user experiences events, is subsequently increased as a result of those events, and then drops back down to the initial pressure.

As used below to describe steps 506A-506C, the terms "subsequent portion of a sleep session," "subsequent portion of the sleep session," "subsequent portion," and "subsequent sleep session portion" can generally refer to one or both of (i) one or more subsequent portions of the current sleep session that follows the first portion of the current sleep session, and (ii) one or more portions of a subsequent sleep session that follows the current sleep session. Similarly, the terms "modified portion of a sleep session," "modified portion of the sleep session," "modified portion," and "modified sleep session portion" generally refers to any portion(s) of any sleep session that is modified in some manner, whether that portion(s) is (i) a subsequent portion of the current sleep session that follows the first portion of the current sleep session, or (ii) a portion of a subsequent sleep session that follows the current sleep session.

At step 506A of method 500, the first pressure in the predetermined range can be modified for the subsequent sleep session portion. When the respiratory therapy system is supposed to supply pressurized air to the user at the first pressure in the predetermined range of pressures (for example, later during the current sleep session, during a subsequent sleep session once the system detects that the user has fallen asleep, or later during the subsequent sleep session), the respiratory therapy system will instead supply the pressurized air to the user at a modified first pressure. In some implementations, modifying the first pressure of the predetermined range of pressures includes increasing the first pressure, such that the modified first pressure is greater than the unmodified first pressure.

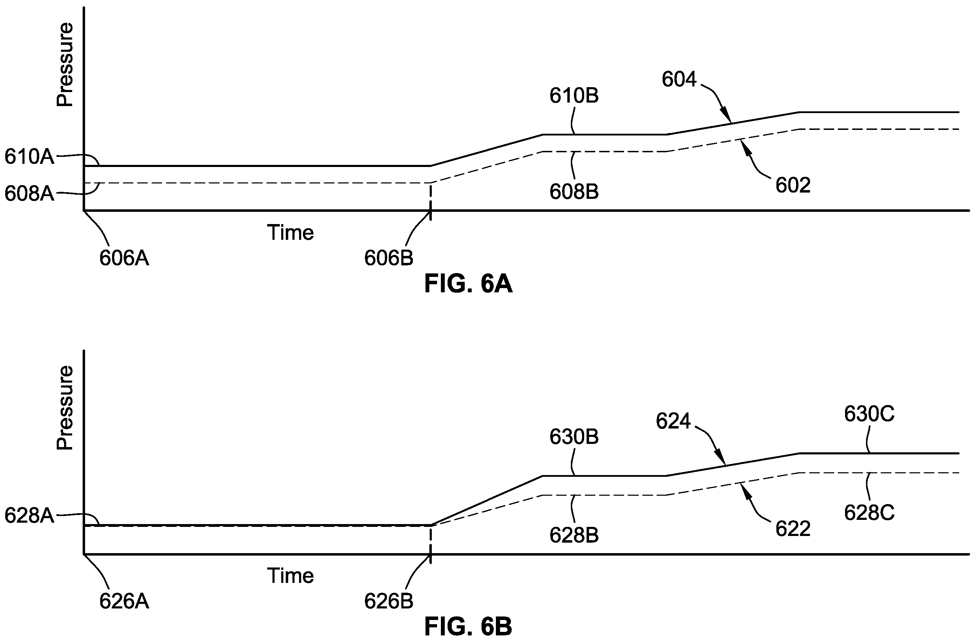

FIG. 6A is pressure versus time plot that shows a pressure curve 602 for an unmodified portion of the current sleep session (dashed line), and a pressure curve 604 for a subsequent sleep session portion that has been modified (solid line). In the subsequent sleep session portion, the pressure has been modified according to step 506A. The pressure curves represent the pressure of the pressurized air that is supplied to the user during the respective sleep session portions. The pressure curve 602 for the unmodified portion of the current sleep session begins at a first pressure 608A and at a first time 606A. The first time 606A may correspond to a time at which the user has initially fallen asleep during the current sleep session, or any time at which the first pressure 608A is supposed to be applied during the current sleep session. At a second time 606B, the pressure curve 602 begins to increase to a second pressure 608B. As can be seen by comparing pressure curves 602 and 604, the pressure of the pressurized air during the subsequent sleep session portion (e.g., during the subsequent portion of the current sleep session and/or during a portion of a subsequent sleep session) is increased relative to the unmodified portion.

In this manner, the pressure curve 604 for the subsequent sleep session portion begins at the first time 606A with a modified first pressure 610A that is greater than the first pressure 608A of the unmodified portion of the current sleep session. Initiating the subsequent sleep session portion by supplying the pressurized air at the modified first pressure 610A instead of the first pressure 608A can aid in decreasing the number, type, and/or severity of events experienced by the user during the subsequent sleep session portion, and/or in later sleep session portions.

In the illustrated implementation, the pressure during the subsequent sleep session portion is greater than the pressure during the unmodified portion for all pressures within the predetermined range of pressures. Thus, at the second time 606B, the pressure curve 604 begins to increase to a modified second pressure 610B that is greater than the second pressure 608B of the pressure curve 602. However, in other implementations, the pressure during the subsequent sleep session portion may start at a modified first pressure that is greater than the first pressure of the current sleep session, but afterwards increases to the pressures in the predetermined range of pressures (e.g., increases to the same pressures as the unmodified portion, in a step-wise or continuous manner). In such implementations, after starting at the modified first pressure 610A during the subsequent sleep session portion, the pressure may increase to a combination of (i) pressures within the predetermined range of pressures and (ii) modified pressures within the predetermined range of pressures. For example, the pressure could begin at the modified first pressure 610A and increase to the second pressure 608B, and then subsequently increase to some pressure greater than the second pressure 608B. This pressure could be a modified second pressure (e.g., a pressure greater than the second pressure from the unmodified portion but less than a third pressure that would be used in the unmodified portion), a third pressure (e.g., the third pressure from the unmodified portion), a modified third pressure (e.g., a pressure greater than the third pressure from the unmodified portion), or some other pressure.

Referring back to FIG. 5, in some implementations, step 506A of method 500 can include determining a minimum pressure for the first portion of the current sleep session at which the event thresholds are not satisfied. The first pressure for the subsequent sleep session portion can then be set to this minimum pressure. In some implementations, the minimum pressure is the minimum pressure at which the total number of events during the first portion of the current sleep session does not satisfy a threshold number of events (e.g., the number of events during the first portion of the current sleep session is less than the threshold number of events). In other implementations, the minimum pressure is the minimum pressure at which the average number of events per a predetermined period of time does not satisfy a threshold average number of events for that period of time. In these implementations, the predetermined period of time can be one minute, two minutes, three minutes, four minutes, five minutes, ten minutes, thirty minutes, one hour, or other periods of time. In still other implementations, the minimum pressure is the minimum pressure at which the total number of a certain type of events (e.g., only obstructive sleep apneas) or the average number of a certain type of event per a predetermined period of time do not satisfy threshold values. In further implementations, the minimum pressure is the minimum pressure at which no events of a certain severity occur. In even further implementations, the minimum pressure is the minimum pressure at which the total number of events of a certain severity or the average number of events of a certain severity do not satisfy threshold values. In any of these implementations, the modified first pressure 610A during the subsequent sleep session portion can be equal to or greater than this minimum pressure.

Step 506B of method 500 shows an additional or alternative step that can be taken when the number, type, and/or severity of events experienced during the first portion of the current sleep session satisfies a threshold. In step 506B, the difference in pressure between the first pressure and the second pressure of the predetermined range can be modified for the subsequent sleep session portion. In some implementations, this modifying can include increasing the second pressure in the subsequent sleep session portion (e.g., to a modified second pressure). In these implementations, the pressure in the subsequent sleep session portion can begin at the same first pressure as the first portion of the current sleep session, but will then increase to a higher second pressure as compared to the first portion of current sleep session.

FIG. 6B is pressure versus time plot that shows a pressure curve 622 for an unmodified portion of the current sleep session (dashed line), and a pressure curve 624 for a subsequent sleep session portion that has been modified (solid line). In the subsequent sleep session portion, the pressure has been modified according to step 506B. The pressure curves can represent the pressure of the pressurized air that is supplied to the user during the respective sleep session portions. Similar to the pressure curve 602 in FIG. 6A, the pressure curve 622 for the unmodified portion begins at a first pressure 628A and at a first time 626A. The first time 626A may correspond to a time at which the user has initially fallen asleep during the current sleep session, or any time at which the first pressure 628A is supposed to be applied. At a second time 626B, the pressure curve 622 for the unmodified portion begins to increase to a second pressure 628B.

The pressure curve 624 for the subsequent sleep session portion begins at the first time 626A with the same first pressure 628A as the pressure curve 622, and at the second time 626B, the pressure curve 624 also begins to increase. However, the pressure curve 624 increases up to a modified second pressure 630B that is greater than the second pressure 628B of the pressure curve 622. Thus, in the subsequent sleep session portion, the difference between the first pressure and the second pressure of the predetermined range of pressures is modified, by increasing the second pressure to a modified second pressure. Increasing the pressure of the pressurized air from the first pressure 628A to the modified second pressure 630B can aid in decreasing the number, type, and/or severity of events experienced by the user during the subsequent sleep session portion, and/or in later sleep session portions.

In the illustrated implementation, after the pressure curve 622 rises to the second pressure 628B, the pressure curve 622 remains constant and then later increases to a third pressure 628C. Similarly, after the pressure curve 624 rises to the modified second pressure 630B, the pressure curve 624 remains constant and then later increases to a modified third pressure 630C. In this manner, only the difference between the first two pressures in the predetermined range of pressures for the subsequent sleep session portion is modified as compared to the unmodified portion (e.g., the difference between the modified second pressure 630B and the modified third pressure 630C is the same as the difference between the second pressure 628B and the modified third pressure 628C). However, in other implementations, the differences between other pressures within the predetermined range of pressures can also be modified for the subsequent sleep session portion (e.g., the difference between the modified second pressure 630B and the modified third pressure 630C can be larger or smaller than the difference between the second pressure 628B and the modified third pressure 628C).

Referring back to FIG. 5, step 506C of method 500 shows an additional or alternative step that can be taken when the number, type, and/or severity of events experienced during the first portion of the current sleep session satisfies a threshold. In step 506C, the rate of change between the first pressure and the second pressure in a subsequent sleep session portion can be modified. In some implementations, this modification includes modifying the amount of time that the pressurized air is supplied at the first pressure in the subsequent sleep session portion. In other implementations, this modification includes modifying the time it takes to increase from the first pressure to the second pressure in the subsequent sleep session portion, once the pressure begins to increase from the first pressure. In further implementations, this modification can include both modifying the amount of time that the pressurized air is supplied at the first pressure in the subsequent sleep session portion, and modifying the time it takes to increase from the first pressure to the second pressure in the subsequent sleep session portion. The modifications can also include decreasing the amount of time that the pressurized air is supplied at the first pressure, decreasing the amount of time it takes to increase from the first pressure to the second pressure, or both.

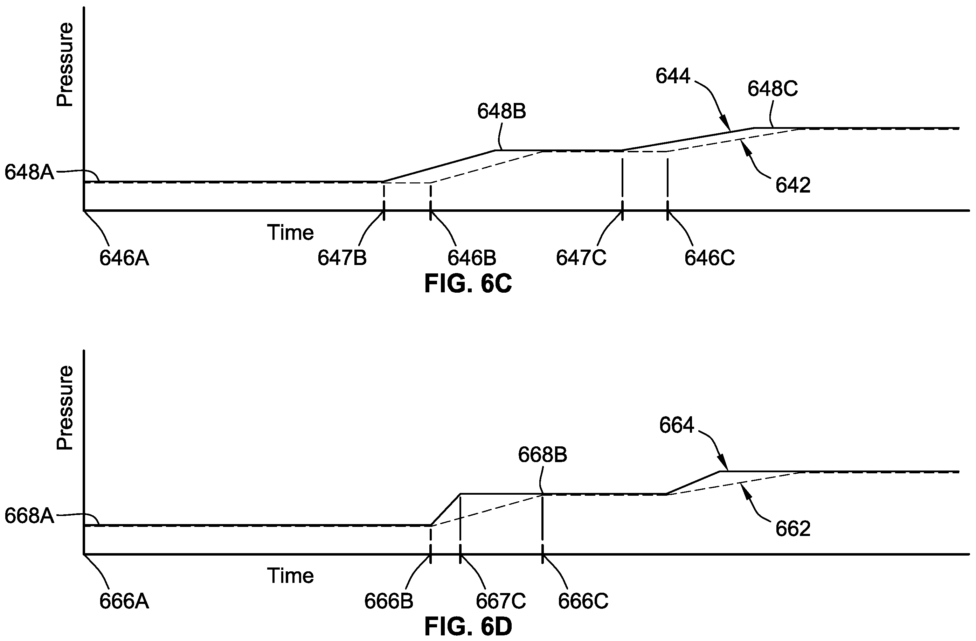

FIG. 6C is pressure versus time plot that shows a pressure curve 642 for an unmodified portion of the current sleep session (dashed line), and a pressure curve 644 for a subsequent sleep session portion that has been modified according to step 506C (solid line). Pressure curve 644 represents implementations where the amount of time that the pressurized air is supplied at the first pressure during the subsequent sleep session portion is modified. Similar to the pressure curve 602 in FIG. 6A and pressure curve 622 in FIG. 6B, the pressure curve 642 for the unmodified portion begins at a first pressure 648A and at a first time 646A. The first time 646A may correspond to a time at which the user has initially fallen asleep during the current sleep session, or any time at which the first pressure 648A is supposed to be applied during the current sleep session. At a second time 646B, the pressure curve 642 begins to increase to a second pressure 648B. Thus, the pressure curve 642 begins to increase from the first pressure 648A to the second pressure 648B after a first time period has elapsed. The first time period is equal to the difference between the first time 646A and the second time 646B.

The pressure curve 644 for the subsequent sleep session portion begins at the first time 646A with the same first pressure 648A as the pressure curve 642, and also eventually increases to the same second pressure 648B. However, as shown in FIG. 6C, while the pressure curve 642 begins to increase from the first pressure 648A at the second time 646B, the pressure curve 644 begins to increase from the first pressure 648A at a modified second time 647B that occurs prior to the second time 646B. Thus, the pressure curve 644 begins to increase from the first pressure 648A to the second pressure 648B after a modified first time period has elapsed. The modified first time period is equal to the difference between the first time 646A and the modified second time 647B.

Thus, the modified first time period for the modified sleep session portion is less than the first time period for the current sleep session. In this manner, during the subsequent sleep session portion, the respiratory therapy system will supply the pressurized at the first pressure of the predetermined range of pressures for a shorter amount of time, which can aid in decreasing the number, type, and/or severity of events experienced by the user during the subsequent sleep session portion, and/or in later sleep session portions.

In some implementations, the amount of time spent applying the first pressure during the subsequent sleep session portion can be determined by selecting a value for the modified first time period that is less than the first time period of the current sleep session. In other implementations, the amount of time spent at the first pressure is based on the number of events experienced during the first portion of the sleep session. During the current sleep session, the pressure can begin to increase from the first pressure to the second pressure after a threshold number of events occur. If event thresholds are met during the first portion of the current sleep session (e.g., if the user experiences too many events, too severe of events, or a combination of both), then the threshold number of events that trigger the increase from the first pressure to the second pressure during the subsequent sleep session portion can be modified to be a modified threshold number of events. This modified threshold number of events to trigger the increase to the second pressure during the subsequent sleep session portion will generally be less than the threshold number of events to trigger the increase to the second pressure during the first portion of the current sleep session. In that case, the pressure will generally increase from the first pressure to the second pressure sooner during the subsequent sleep session portion as compared to the first portion of the current sleep session, and thus the rate of change between the first pressure and the second pressure is modified.

As shown in FIG. 6C, the pressure curve 642 subsequently begins to increase from the second pressure 648B to a third pressure 648C at a third time 646C. Thus, the pressure curve 642 begins to increase from the second pressure 648B after a time period has elapsed that is equal to the difference between the second time 646B and the third time 646C. Similarly, the pressure curve 644 subsequently beings to increase from the second pressure 648B to the third pressure 648C. However, the pressure curve 644 begins to increase from the second pressure 648B at a modified third time 647C that occurs before the third time 646C. Thus, the pressure curve 644 begins to increase from the second pressure 648B to the third pressure 648C after a time period has elapsed that is equal to the difference between the second time 646B and the modified third time 647C. In some implementations, the difference between the modified second time 647B and the modified third time 647C is equal to or less than the difference between the second time 646B and the third time 646C.

FIG. 6D is pressure versus time plot that shows a pressure curve 662 for an unmodified portion the current sleep session (dashed line), and a pressure curve 664 for a subsequent sleep session portion that has been modified according to step 506C (solid line). Pressure curve 664 represents implementations where the amount of time it takes for the pressurized air to increase from the first pressure to the second pressure during the subsequent sleep session portion is modified. Similar to the pressure curves 602 (FIG. 6A), 622 (FIG. 6B), and 642 (FIG. 6C), the pressure curve 662 for the current sleep session begins at a first pressure 668A and at a first time 666A. The first time 666A may correspond to a time at which the user has initially fallen asleep during the current sleep session, or any time at which the first pressure 668A is supposed to be applied. The pressure curve 662 begins to increase to a second pressure 668B at a second time 666B, and reaches the second pressure 668B at a third time 666C. Thus, the pressure curve 662 begins to increase from the first pressure 668A to the second pressure 668B after a first time period has elapsed that is equal to the difference between the first time 666A and the second time 666B. For the pressure curve 662 in the unmodified portion of the current sleep session, a second time period elapses between (i) the second time 666B when the pressure curve 642 begins to increase from the first pressure 648A, and (ii) the third time 666C when the pressure curve 642 reaches the second pressure 668B.

The pressure curve 664 for the subsequent sleep session portion starts at the same first pressure 668A and the same first time 666A as the pressure curve 662. The pressure curve 664 also begins to increase from the first pressure 668A to the second pressure 668B at the second time 666B, after the first time period has elapsed. However, the pressure curve 664 reaches the second pressure 668B at a modified third time 667C, which occurs before the third time 666C at which the pressure curve 662 reaches the second pressure 668B. Thus, for pressure curve 664 in the subsequent sleep session portion, a modified second time period elapses between (i) the second time 666B when the pressure curve 644 begins to increase from the first pressure 668A, and (ii) the modified third time 667C when the pressure curve 644 reaches the second pressure 668B. The modified second time period for the subsequent sleep session portion is thus less than the second time period for the unmodified portion of the current sleep session. In this manner, during the subsequent sleep session portion, the respiratory therapy system will increase more quickly to the second pressure of the predetermined range of pressures, which can aid in decreasing the number, type, and/or severity of events experienced by the user during the subsequent sleep session portion, and/or in later sleep session portions.

FIGS. 6A-6D illustrate a number of ways that the pressure of the pressurized air can be modified during a subsequent sleep session portion to aid in decreasing the number of events, the number of certain types of events, or the number of events of a certain severity, experienced by the user during the subsequent sleep session portion, and/or in later sleep session portions. The pressurized air during the subsequent sleep session portion can begin at a higher pressure.

The pressurized air during the subsequent sleep session portion can also increase to higher pressures at a faster rate (e.g., by staying at lower pressures for shorter time, and/or by taking less time to increase to higher pressures once the pressure increase begins). The pressurized air during the subsequent sleep session portion can also start at the same initial pressure, but when increasing from that initial pressure, the increase is to a higher pressure than otherwise dictated by the pressure curve for the current sleep session. In some implementations, the pressurized air for the subsequent sleep session portion can be modified in other ways as well. For example, the pressurized air could begin at a lower pressure, the difference between the first pressure and the subsequent pressures could decrease, the rate of change from the first pressure to the second pressure can decrease, or any combination thereof could occur.

These modifications may be desired if the number, type, and/or severity of events detected during the subsequent sleep session portion satisfy a predetermined threshold for the subsequent sleep session portion, or satisfy a predetermined threshold within a predetermined period of time. In still other implementations, multiple modifications can occur. For example, the pressurized air for the subsequent sleep session portion may initially be supplied at the modified first pressure that is greater than the first pressure of the predetermined range of pressures, and the pressurized air can increase to higher pressures at a faster rate.

In some implementations, data from one or more prior sleep sessions can be received and analyzed to aid in determining how to modify the pressure during the subsequent sleep session portion (e.g., a subsequent portion of the current sleep session or a portion of a subsequent sleep session). Instead of only modifying the pressurized air for a subsequent sleep session portion based on whether the number of events (or the type and/or severity of events) satisfies a threshold in the first portion of the current sleep session, events experienced during one or more prior sleep sessions can be taken into account. For example, in some implementations, the pressurized air during the subsequent sleep session portion is only modified if an average number of events was experienced during the first portion of the current sleep session and one or more prior sleep sessions (or one or more individual portions of the one or more prior sleep session), instead of only the first portion of the current sleep session.

In some implementations, the modification of the pressurized air supplied to the user by the respiratory therapy system during the subsequent sleep session portion can be based on data from the current sleep session, and activities that the user participated in the day preceding the current sleep session. Activities such as exercise, an increased or decreased amount of exercise compared to a baseline, consumption of alcohol, an increased or decreased consumption of alcohol compared to a baseline, consumption of caffeine, an increased or decreased consumption of caffeine compared to a baseline, consumption of food, and/or an increased or decreased consumption of food compared to a baseline, can all negatively affect the number, type, and/or severity of events experienced by the user during the initial portions of sleep sessions immediately following those activities. Such activities can be self-reported and/or recorded by an activity tracker, such as activity tracker 190. Activity data can be combined with event data generated by the activity tracker, or pressure and/or flow rate sensors, such as pressure sensor 132 and flow rate sensor 134, and the effect of the activity on the number, type, and/or severity of events determined. If the user participates in these activities and then suffers from an abnormally large number of events (or an abnormally large number of events of a certain type or severity) during the first portion of the current sleep session, the pressurized air during the subsequent sleep session portion (e.g., a subsequent portion of the current sleep session or a portion of a subsequent sleep session) can be modified to a lesser degree than would otherwise have taken place had the user not participated in those activities. Similarly, if the user participates in activities known to positively affect the number, type, and/or severity of events experienced by the user during the initial portions of sleep sessions immediately following those activities, the pressurized air for the subsequent sleep session portion can be modified more than it otherwise would be had the user not participated in those activities.

In some implementations, data from one or more prior sleep sessions can be received and analyzed to aid in determining whether to use that data (or other data associated with those prior sleep sessions) to aid in determining how to modify the pressurized air during the subsequent sleep session, based on activities participated in during those prior sleep sessions. Thus, method 500 can include identifying a first set of one or more prior sleep sessions that occurred following activities known to cause the user to experience (i) more events than typical during the next sleep session, (ii) more severe events than typical during the next sleep session, or (iii) both (i) and (ii); and then discarding the data from the first set of one or more prior sleep sessions.

Similarly, method 500 can also include identifying a second set of one or more prior sleep sessions that did not occur after any of these undesirable activities, and retaining the data from the second set of one or more prior sleep sessions. In this manner, in some implementations, any modifications to the pressurized air supplied to the user by the respiratory therapy system during the subsequent sleep session portion can specifically not be based on data from the first set of prior sleep sessions, and can instead be based only on (i) data associated with the current sleep session, (ii) data associated with the second set of prior sleep sessions, or (iii) both (i) and (ii).

In some implementations, method 500 can include transmitting a notification to the user or to a third party, that the pressure of the pressurized air is to be modified for the subsequent sleep session portion. For example, if the predetermined range of pressures is prescribed by the user's healthcare provider, the system can transmit a notification to the user's healthcare provider indicating that the predetermined range of pressures was not sufficient (i) to prevent the user from experiencing unwanted numbers or types of events during the first portion of the current sleep session (and/or other early portions of the current sleep session), or (ii) to reduce the amount or types of events to a desired level. This notification can be transmitted to the user and/or to other third parties as well, such as a family member of the user, friend of the user, and/or a caregiver of the user.

In some implementations, the modification of the pressurized air during the subsequent sleep session portion can be based on the type of events experienced by the user during the current sleep session. For example, hypopneas are partial occlusions of the user's airway, while obstructive apneas are full obstructions of the user's airway. If the user experiences a large number of hypopneas but not obstructive apneas, the pressurized air may be modified to a lesser degree for the subsequent sleep session portion than the pressurized air otherwise would be modified if the user was experiencing obstructive apneas. In still other implementations, the modification of the pressurized air during the subsequent sleep session portion can be based on the type of events experienced by the user during the subsequent sleep session portion. For example, if it is determined that the user is suffering from obstructive apneas during the subsequent sleep session portion, the system can modify the pressurized air for that subsequent sleep session portion to reduce the number of obstructive apneas that are occurring (for example by increasing faster to higher pressures). However, if the user is only experiencing hypopneas, the system can be configured to not modify the pressurized air, and instead increase the pressurized air according to the original determined pressure increases.

In some implementations, the modification of the pressurized air during the subsequent sleep session portion can be based on when events occur relative to increases in the pressurized air. For example, if during the current sleep session the user experiences an event shortly after the pressure of the pressurized air increases, the system can decide to increase the pressure during a subsequent sleep session portion more rapidly, and/or to increase the pressure during the subsequent sleep session portion to a higher pressure. Similarly, if during the current sleep the user does not experience any events following a pressure increase, the system can decide to maintain the pressure during a subsequent sleep session portion for a longer period of time. This temporal aspect can also be used during the subsequent sleep session portion for an "on-the-fly" (e.g., real-time) modification of the pressurized air during the subsequent sleep session portion.

Generally, a number of different types of event thresholds can be used. In some implementations, the event threshold is a threshold number of events, a threshold number of events of a certain type, a threshold number of events of a certain severity, or any combination thereof. In other implementations, the event threshold is a threshold average number of events, a threshold average number of events of a certain type, a threshold average number of events of a certain severity, or any combination thereof. The average can be an average per unit of time, which can be per minute, per hour, etc. The average can also be an average per unit of time spent asleep, which can be per minute spent asleep, per hour spent asleep, etc. The thresholds can be based only on events occurring during the current sleep session, only on events occurring during one or more prior sleep session, or both on events occurring the during current sleep session and events occurring during one or more prior sleep sessions. Thus, for example, the threshold average number of events (or average number of events of a certain type and/or severity) can be a threshold average across multiple sleep sessions.

In one non-limiting example, the threshold number of events is one event, two events, three events, four events, five events, or ten events. In another non-limiting example, obstructive apneas and hypopneas can count towards threshold number of events, and central apneas, snores, and other minor flow limitations do not count toward the threshold number of evens. In a further non-limiting example, the events can be weighted by severity when counting the number of events. In this non-limiting example, a severity index can be determined that weights the severity based on the types, duration, and timing of the events, and the threshold is a threshold severity index. The severity index can be given by the following equation:

$$Severity\ Index = \frac{1}{T}\sum_{n=1}^{N}\left(w_1 event_{type} + w_2 event_{duration} + \frac{w_3}{time\_since\_last\_event}\right),$$

where $T$ is the duration spent at a given pressure range; $N$ is the number of events that occur when in the given pressure range; event_type is a number value representing the severity of each event based on the type of the event (1 for an obstructive apnea or a hypopnea, 0.5 for a snore, 0.25 for a flow limitation, and 0 for a central apnea), event_duration is a number value representing the duration of each event, time_since_last_event is a number value representing the time since the last event occurred for each event, and $w_1$, $w_2$, and $w_3$ are the weights given to those values. In this example, multiple events occurring in short succession are given more weight than events spread out over time.

The thresholds can also be based on events occurring more than just the first portion of the current sleep session (e.g., the portion of the current sleep session when the pressurized air is supplied at the first pressure of the predetermined range of pressures). The thresholds can be based on the first two portions (e.g., the first pressure and the second pressure of the predetermined range of pressures), the first three portions (e.g., the first pressure, the second pressure, and the third pressure of the predetermined range of pressures), the first five portions (e.g., the first pressure through the fifth pressure of the predetermined range of pressures), etc. Thus, for example, the threshold average number of events (or average number of events of a certain type and/or severity) can be a threshold average across a plurality of portions of the current sleep session.

Similarly, while FIGS. 6A-6D generally show modifications to the first portion of the subsequent sleep cycle, the modifications can additionally or alternatively include modifications to other portions of the sleep cycle. For example, the first portion of the subsequent sleep cycle can be modified along with the second portion and the third portion, in order to reduce the number and/or severity of events occurring during those portions of the subsequent sleep cycle.

In some implementations, the threshold can be a weighted average that is weighted based on the pressure of the pressurized air during each event (in the first portion of the current sleep session, or in one or more additional portions of the current sleep session and/or one or more prior sleep sessions). In some cases, events that occur at higher pressures are weighted more heavily than events that occur at lower pressures (e.g., events occurring at higher pressure count more toward determining whether the relevant threshold has been satisfied).

In other cases, the events could be weighted based on different factors. For example, events of a certain type (such as obstructive apneas) can be weighted more heavily than events of another type (such as hypopneas). In another example, events of a greater/higher severity can be weighted more heavily than events of a lesser/lower severity. In a further example, if it is suspected that the user suffers from positional SDB (e.g., positional OSA) and/or sleep-stage dependent SDB (e.g., REM-dominant SDB such as REM-dominant OSA), events occurring when the user is in certain body positions and/or certain sleep stages can be weighted more or less heavily. In an even further example, events can be weighted more heavily if they occur in a cluster with other events (e.g., two events that occur 1 minute apart may be weighted more heavily than two events that occur 10 minutes apart, even if the events are otherwise equal (based on type, severity, body position, sleep stage, etc.)).

In further implementations, the threshold is an index that is calculated based on the types of events, the duration of events, the time since the last event, and/or any other factors. This index can be similar to the severity index discussed above, and can weight various different factors or values of factors differently, depending on how the factors affect the sleep session.

Generally, the modifications discussed herein with respect to FIG. 5 and FIGS. 6A-6D, and any related features, can be made to any pressure or set of pressures in the subsequent sleep session portion. For example, regardless of which pressure of the predetermined range of pressures is being supplied, the next pressure can be modified, the rate at which the pressure is increased to the next pressure can be modified, the difference between any two pressures can be modified, or any combination of these modifications can occur.

The modifications can also be made contingent on other factors. For example, if the user suffers from positional SDB or sleep-stage dependent SDB, the pressure settings during the subsequent sleep session portion can be made only if it is determined that the user is in a body position that tends to lead to more events, and/or if the user is in a sleep stage that tends to lead to more events. Thus, even in the number, type, and/or severity of events satisfy the event threshold(s) during the first portion of the current sleep session, modifications to the pressure settings may only be made during the subsequent sleep session portion if the user is in a disadvantageous body position and/or a disadvantageous sleep stage. In other words, if the user is in an advantageous body position and/or sleep stage, no modifications are made, even if the event thresholds are satisfied. The converse may also be true. If the number, type, and/or severity of the events during the first portion of the current sleep session are sufficiently close to satisfying the event thresholds, one or more modifications can be made during the subsequent sleep session portion if it is determined that the user is in a disadvantageous body position and/or sleep stage.

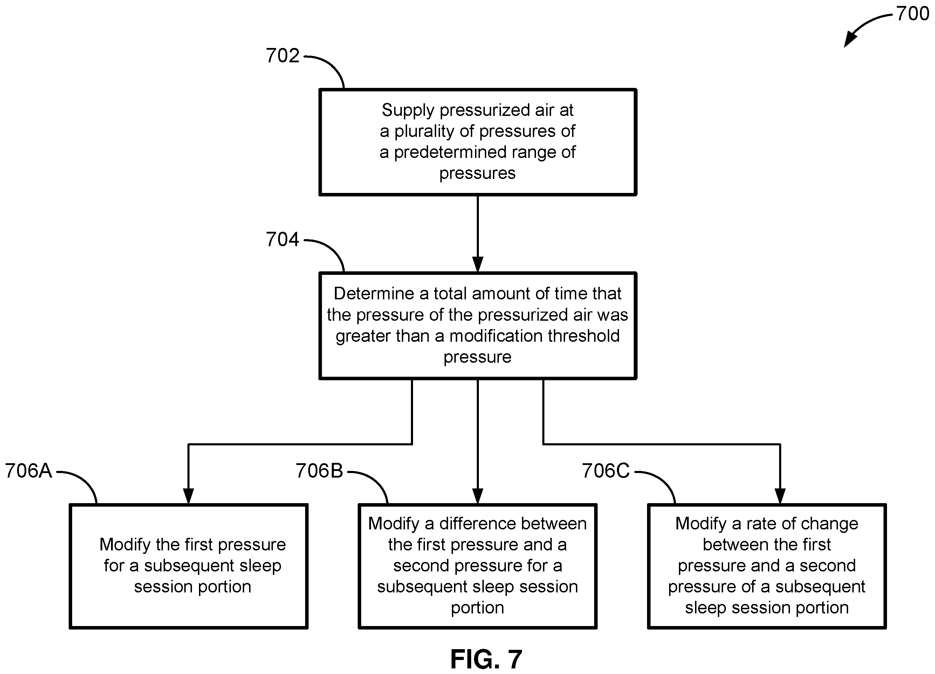

Referring to FIG. 7, a method 700 for modifying pressure settings of a respiratory therapy system (such as respiratory therapy system 120) is illustrated. In some implementations, the respiratory therapy system includes a respiratory therapy device configured to supply pressurized air (such as respiratory therapy device 122), and a user interface (such as user interface 124) coupled to the respiratory therapy device via a conduit (such as conduit 126). The user interface is configured to engage with the user, and aids in directing the pressurized air to the user's airway. Generally, a control system having one or more processors (such as control system 110 of system 100) is configured to carry out the steps of method 700. The control system can be coupled to a memory device (such as memory device 114 of system 100) that stores machine-readable instructions. The machine-readable instructions can be executed by at least one of the one or more processors of the control system to carry out the steps of method 700. The memory device can also store any type of data utilized in the steps of method 700. Generally, method 700 can be implemented using a system (such as system 100) that includes the respiratory therapy system, the control system, and the memory device. Method 700 can also be implemented using a computer program product (such as a non-transitory computer readable medium) comprising instructions that when executed by a computer, cause the computer to carry out the steps of method 700.

Method 700 is similar to method 500, and illustrates a technique for modifying the pressure settings of the respiratory therapy system for a subsequent sleep session portion (e.g., a subsequent portion(s) of the current sleep session or a portion(s) of a subsequent sleep session) if it is determined that the settings were not satisfactory for the first portion of the current sleep session, or for the entire current sleep session. However, instead of monitoring events experienced during the first portion (and/or other portions) of the current sleep session, method 700 involves tracking the amount of time during the current sleep session at which the pressure of the pressurized air was greater than a modification threshold pressure. If it is determined after the end of the current portion of the current sleep session (and/or after the end of the entirety of the current sleep session) that the pressure was above this threshold for a greater amount of time than desired, the settings of the respiratory therapy system can then be modified for the subsequent sleep session portion.

Step 702 of method 700 includes supplying pressurized air to a user during a current sleep session. Step 702 of method 700 is similar to step 502 of method 500, and thus the pressurized air is supplied according to a predetermined set or range of pressures that may be prescribed by the user's healthcare provider. However, in step 702, the pressurized air is supplied at a plurality of pressures within the predetermined range of pressures, which includes at least a first pressure and a second pressure that is greater than the first pressure. In some implementations, the plurality of pressures represents the entire predetermined range of pressures, and thus step 702 encompasses an entire sleep session. In other implementations, the plurality of pressures represents a portion of the predetermined range of pressures.

Step 704 of method 700 includes determining the total amount of time during the current portion of the current sleep session or the entirety of the current sleep session that the pressure of the pressurized air is greater than the modification threshold pressure. In some implementations, the modification threshold pressure is about 10 cmH$_2$O. In some implementations, step 704 occurs after the current sleep session (or the current portion of the sleep session) has ended, and thus the amount of time determined at step 704 is an amount of time across all of the current sleep session (or all of the current portion of the current sleep session). The amount of time can be determined as an absolute time, (e.g., the pressure was greater than the threshold pressure for X minutes or Y hours), or as a relative time. The relative time can be a percentage of the overall time of the entire current sleep session (e.g., the pressure was greater than the threshold pressure for Z% of the sleep session). When the amount of time is determined as a relative time, the amount of time is generally determined relative to the total amount of time that the user was actually receiving respiratory therapy, e.g., using the respiratory therapy device 122 to deliver pressurized air through the user interface 124 and the conduit 126. The total amount of time that the user receiving respiratory therapy during the sleep session can be referred to as the therapy session.

In some implementations, the sleep session lasts longer than the therapy session. In these implementations, the sleep session includes periods of time where the user is not receiving respiratory therapy for some reason. Thus, the amount of time spent above the modification threshold pressure in these implementations is generally only determined relative to the total amount of time that the user was receiving pressurized air (the therapy session), and not the total amount of time that the sleep session lasted.

However, in other implementations, the sleep session and the therapy session may be the same length, as the user received respiratory therapy for the entire sleep session. Thus, the amount of time spent above the modification threshold pressure in these other implementations is determined relative to the total amount of time that the user was receiving any pressurized air at all and the amount of time of the sleep session, because those will be the same length of time.

Steps 706A, 706B, and 706C illustrate different actions that can be taken in response to determining that the amount of time during the current sleep session that the pressure is greater than the modification threshold pressure, is greater than or equal to a modification threshold amount of time. As discussed herein, the pressure of the pressurized air during the current sleep session can be based at least in part on events occurring during the current sleep session. If the user experiences events, the pressure of the pressurized air may be increased more quickly during the current sleep session than would otherwise occur if the user experienced little to no events. Thus, if the total amount of time spent above the modification threshold pressure is greater than or equal to the modification threshold amount of time, this indicates that the initial pressure (or the first few pressures) of the predetermined range of pressures may not be sufficiently high to prevent events from occurring. In implementations where the total of amount of time is determined as an absolute time, the modification threshold amount of time can be about three hours, about four hours, about five hours, about six hours, about seven hours, or about eight hours. In implementations where the total amount of time is determined as a percentage of the current sleep session, the modification threshold amount of time can be about 50%, about 60%, about 70%, about 80%, or about 90%.

Step 706A of method 700 is similar to step 506A of method 500, and includes modifying the first pressure for a subsequent sleep session portion (e.g., a subsequent portion(s) of the current sleep session or a portion(s) of a subsequent sleep session). Such a modification can include modifying the first pressure to be a modified first pressure for the subsequent sleep session portion. The modified first pressure for the subsequent sleep session portion may be greater than the first pressure for the current sleep session. Generally, details discussed herein with respect to step 506A are generally also applicable to step 706A.

In some implementations, the value of the modified first pressure for the subsequent sleep session portion is based on the time spent at various pressures during the current sleep session. After the current sleep session is complete (and/or after the current portion of the current sleep session is completed), data from the current sleep session can be analyzed to determine a minimum threshold pressure. This minimum threshold pressure can represent a pressure at which the time spent below that pressure satisfies some minimum threshold amount of time. In some of these implementations, the amount of time during the current sleep session spent below the minimum threshold pressure is less than the minimum threshold amount of time, while the amount of time during the current sleep session spent equal to or above the minimum threshold pressure is greater than or equal to the minimum threshold amount of time. Thus, the minimum threshold pressure generally represents a pressure that divides the entire time of the current sleep session (or current portion of the current sleep session) into a first percentage and a second percentage. The first percentage and the second percentage add up to the entire time of the current sleep session, and the second percentage is generally greater than the first percentage.

In one example, the first pressure for the current sleep session is about 4 $cmH_2O$, the minimum threshold pressure is about 7 $cmH_2O$, and the minimum threshold amount of time is about 5% of the total time of the current sleep session. Thus, in this example, 5% of the current sleep session (or current portion of the current sleep session) is spent below 7 $cmH_2O$, and the first percentage is 5%. Correspondingly, 95% of the current sleep session (or current portion of the current sleep session) is spent at or above 7 $cmH_2O$, and the second percentage is 95%. For the subsequent sleep session portion, the modified first pressure can be 7 $cmH_2O$.

This technique can also be used to adjust the modification threshold pressure which triggers the modifications for the subsequent sleep session portion. For example, the modification threshold pressure can initially be set at 10 $cmH_2O$, and the pressure settings for one or more subsequent sleep session portions can be modified. However, if it is revealed that the first 5% of the time of these subsequent sleep session portions ends at a lower pressure (such as 7 $cmH_2O$), the modification threshold pressure can then be lowered for further subsequent sleep session portions.

Step 706B of method 700 is similar to step 506B of method 500, and includes modifying the difference between the first pressure and the second pressure for the subsequent sleep session portion. Such a modification can include modifying the second pressure for the subsequent sleep session portion to be a modified second pressure, which may be greater than the first pressure for the current sleep session, the second pressure for the current sleep session, or both. Generally, details discussed herein with respect to step 506B are generally also applicable to step 706B.

Step 706C of method 700 is similar to step 506C of method 500, and includes modifying the rate of change between the first pressure and the second pressure for the subsequent sleep session portion. Such a modification can include modifying the time it takes the pressure to begin to increase from the first pressure to the second pressure, modifying the time it takes the pressure to reach the second pressure once the pressure begins to increase from the first pressure, or both. These modifications can include decreasing these times.

In some implementations, these modifications in steps 706A, 706B, and/or 706C can be based further on data from prior sleep sessions, similar to steps 506A, 506B, and/or 506C, in order to improve the accuracy of the analysis. Further, data from certain prior sleep sessions can be discarded, such that the modification(s) for the subsequent portion does not include data from discarded prior sleep session, and/or include only data from the current sleep session and non-discarded prior sleep sessions. The data associated with the prior sleep sessions can be indicative of activities undertaken by the user prior to those prior sleep sessions, and the discarded prior sleep sessions can be sleep sessions that followed consumption of alcohol, food, caffeine, or any combination thereof. In still further implementations, the modifications in steps 706A, 706B, and/or 706C can be based further on activities undertaken by the user prior to the current sleep session.

In some implementations, method 700 can include transmitting a notification to the user or to a third party, that the pressure of the pressurized air is to be modified for the subsequent sleep session portion. For example, if the total amount of time spent at or above the modification threshold pressure during the current sleep session satisfies the modification threshold amount of time, the system can transmit a notification to the user's healthcare provider indicating that the predetermined range of pressures was not sufficient to prevent the user from experiencing elevated pressure levels during the current sleep session. This notification can be transmitted to the user and/or to other third parties as well, such as a family member of the user, friend of the user, and/or a caregiver of the user.

In some implementations, additional or alternative modifications can be made. For example, in some cases the pressure of the pressurize air is increased in response to events experienced during the sleep session. Based on events experienced during a portion of the current sleep session, a subsequent sleep session portion can be modified such that the pressure is increased more quickly following events that are experienced during the subsequent sleep session portion.

Similar to method 500, the modifications discussed herein with respect to method 700 can be affected by the user's body position and/or sleep stage. If the user is in an advantageous body position and/or sleep stage, no modifications may be made during the subsequent sleep session portion even if the modification threshold amount of time is satisfied. Conversely, modifications may be made during the subsequent sleep session portion despite the modification threshold amount of time not being satisfied, if the user is in a disadvantageous body position and/or sleep stage.

In some implementations, modifications to how the pressure of the pressurized air is decreased can be made, in addition to or as an alternative to the modifications discussed above with respect to FIG. 5-7. As noted herein, the pressure of the pressurized air can generally be decreased (for example back to the lowest pressure of the predetermined range of pressures) if the user does not experience any events for a certain amount of time. In these cases, modifications can be made to the decrease in pressure in the subsequent sleep session portion (current sleep session or subsequent sleep session) In one example, the pressure of the pressurized air may not be decreased until it is determined that the user has changed their body position, transitioned to a different sleep stage, or both. In another example, the pressure of the pressurized air is not decreased until a larger amount of time than usual has elapsed since the last event occurred. In a further example, the rate of the decrease in pressure back to the first pressure can be decreased, so that the pressure returns to the first pressure more slowly. In yet another example, the pressure is not decreased until it is determined that the user has changed their body position and/or transitioned to a different sleep stage, even if a sufficient amount of time has elapsed since the last event.

While the present disclosure has been described with reference to one or more particular embodiments or implementations, those skilled in the art will recognize that many changes may be made thereto without departing from the scope of the present disclosure. Each of these implementations and obvious variations thereof is contemplated as falling within the scope of the present disclosure. It is also contemplated that additional implementations according to aspects of the present disclosure may combine any number of features from any of the implementations described herein.

**Claims**

1.    A system (100) comprising:

a respiratory therapy system (120) configured to supply pressurized air at a first pressure of a predetermined range of pressures to a user (210) via the respiratory therapy system (120) during a current sleep session, the predetermined range of pressures further including at least a second pressure greater than the first pressure; a memory (114) storing machine-readable instructions; and a control system (110) including one or more processors (112) configured to execute the machine-readable instructions to:

determine (504) a number of events experienced by the user during a first portion of the current sleep session while the pressurized air is supplied at the first pressure, determining a type of each event experienced by the user during the first portion of the current sleep session, or both; and in response to the number of events, the type of each event, or both, experienced during the first portion of the current sleep session satisfying a threshold, modify (506) (i) the first pressure to be a modified first pressure for a subsequent sleep session portion of the user, such that at a beginning of the subsequent sleep session portion, the pressurized air is supplied to the user via the respiratory therapy (120) system at the modified first pressure, (ii) a difference in pressure between the first pressure and the second pressure of the predetermined range of pressures for the subsequent sleep session portion, (iii) a rate of change from the first pressure to the second pressure of the predetermined range of pressures for the subsequent sleep session portion, or (iv) any combination of (i)-(iii),

wherein during the current sleep session, a pressure of the supplied pressurized air begins to increase from the first pressure to a second pressure after a first time period, **characterized in that**, during the current sleep session, a second time period elapses between (i) the pressure of the supplied pressurized air beginning to increase from the first pressure and (ii) the pressure of the supplied pressurized air reaching a second pressure, and wherein modifying the rate of change from the first pressure to the second pressure for the subsequent sleep session portion includes modifying the second time period for the subsequent sleep session portion to be a modified second time period.

2.    The system of claim 1, wherein:

(a) the subsequent sleep session portion includes one or more subsequent portions of the current sleep session, one or more portions of a subsequent sleep session, or both, and/or the beginning of the subsequent sleep session portion occurs when the user initially falls asleep during the subsequent sleep session portion, when the first pressure in the predetermined range of pressures is applied, or both; or (b) the modified first pressure of the subsequent sleep session portion is greater than the first pressure of the current sleep session.

3.    The system of claim 1 or claim 2, wherein:

the one or more processors of the control system are further configured to execute the machine-readable instructions to determine a minimum pressure at which an average number of events for a predetermined period of time during the first portion of the current sleep session does not satisfy the threshold, the modified first pressure of the subsequent sleep session portion being equal to the minimum pressure; or

the one or more processors of the control system are further configured to execute the machine-readable instructions to determine a minimum pressure at which an average number of events for a predetermined period of time during the first portion of the current sleep session does not satisfy the threshold, the modified first pressure of the subsequent sleep session portion being equal to the minimum pressure; and wherein (i) the minimum pressure is greater than the first pressure of the current sleep session, (ii) the predetermined period of time is one hour; or (iii) both (i) and (ii).

4. The system of any one of claims 1 to 3, wherein:

modifying the difference in pressure between the first pressure and the second pressure for the subsequent sleep session portion includes modifying the second pressure to be a modified second pressure; and/or

modifying the difference in pressure between the first pressure and the second pressure for the subsequent sleep session portion includes modifying the second pressure to be a modified second pressure that is greater than the first pressure, the second pressure, or both.

5. The system of any one of claims 1 to 4, wherein:

modifying the rate of change from the first pressure to the second pressure for the subsequent sleep session portion includes modifying the first time period for the subsequent sleep session portion to be a modified first time period; or

modifying the rate of change from the first pressure to the second pressure for the subsequent sleep session portion includes modifying the first time period for the subsequent sleep session portion to be a modified first time period that is less than the first time period for the current sleep session; or

modifying the rate of change from the first pressure to the second pressure for the subsequent sleep session portion includes modifying the first time period for the subsequent sleep session portion to be a modified first time period that is less than the first time period for the current sleep session, the pressure of the supplied pressurized air in the subsequent sleep session portion being configured to increase from the first pressure to the second pressure after the modified first time period.

6. The system of any one of claims 1 to 5, wherein:

the pressure during the current sleep session is configured to begin to increase from the first pressure to the second pressure after a threshold number of events occur during the first portion of the current sleep session at which the pressurized air is supplied to the user at the first pressure; or

the pressure during the current sleep session is configured to begin to increase from the first pressure to the second pressure after a threshold number of events occur during the first portion of the current sleep session at which the pressurized air is supplied to the user at the first pressure, and wherein modifying the rate of change from the first pressure to the second pressure for the subsequent sleep session portion includes modifying the threshold number of events to be a modified threshold number of events for the subsequent sleep session portion.

7. The system of claim 6, wherein:

the modified threshold number of events for the subsequent sleep session portion is less than the threshold number of events for the current sleep session; or

the modified threshold number of events for the subsequent sleep session portion is less than the threshold number of events for the current sleep session, and wherein the pressure during the subsequent sleep session portion is configured to increase from the first pressure to the second pressure after a modified first time period that is less than the first time period of the current sleep session.

8. The system of any one of claims 1 to 7, wherein:

the modified second time period for the subsequent sleep session portion is less than the second time period for the current sleep session; or

the modified second time period for the subsequent sleep session portion is less than the second time period for the current sleep session, and wherein during the subsequent sleep session portion, the modified second time period elapses between (i) the pressure beginning to increase from the first pressure or the modified first pressure, and (ii) the pressure reaching the second pressure or the modified second pressure.

9. The system of any one of claims 1 to 8, wherein:

the threshold is a threshold average number of events per unit of time for the first portion of the current sleep session and/or for a plurality of portions of the current sleep session; or

the threshold is a threshold average number of events per unit of time for the first portion of the current sleep session; and/or for a plurality of portions of the current sleep session, and the threshold average number of events per unit of time is weighted based on the pressure of the pressurized air during each event, a body position of the user during each event, a sleep stage of the user during each event, or any combination thereof; or

the threshold is a threshold average number of events per unit of time for the first portion of the current sleep session; and/or for a plurality of portions of the current sleep session, and the threshold average number of events per unit of time is weighted based on the pressure of the pressurized air during each event, a body position of the user during each event, a sleep stage of the user during each event, or any combination thereof, and wherein events occurring when the pressurized air is supplied at the second pressure are weighted more heavily than events occurring when the pressurized air is supplied at the first pressure.

10. The system of any one of claims 1 to 9, wherein the threshold is a threshold average number of events per unit of time while the user is asleep during the current sleep session.

11. The system of any one of claims 1 to 10, wherein the one or more processors of the control system are further configured to execute the machine-readable instructions to receive data associated with the current sleep session and data associated with one or more prior sleep sessions of the user, and wherein (i) the modification of the first pressure for the subsequent sleep session portion, (ii) the modification of the difference in pressure between the first pressure and the second pressure for the subsequent sleep session portion, (iii) the modification of the rate of change from the first pressure to the second pressure for the subsequent sleep session portion, or (iv) any combination of (i)-(iii), is based at least in part on the data associated with the one or more prior sleep sessions.

12. The system of claim 11, wherein the one or more processors of the control system are further configured to execute the machine-readable instructions to:

identify a first set of one or more prior sleep sessions of the one or more prior sleep sessions;
identify a second set of one or more prior sleep sessions of the one or more prior sleep sessions; and
discard the data associated with the first set of one or more prior sleep sessions, such that (i) the modification of the first pressure for the subsequent sleep session portion, (ii) the modification of the difference in pressure between the first pressure and the second pressure for the subsequent sleep session portion, (iii) the modification of the rate of change from the first pressure to the second pressure for the subsequent sleep session portion, or (iv) any combination of (i)-(iii), is not based on the data associated with the first set of one or more prior sleep sessions; or

wherein the one or more processors of the control system are further configured to execute the machine-readable instructions to:

identify a first set of one or more prior sleep sessions of the one or more prior sleep sessions;
identify a second set of one or more prior sleep sessions of the one or more prior sleep sessions; and
discard the data associated with the first set of one or more prior sleep sessions, such that (i) the modification of the first pressure for the subsequent sleep session portion, (ii) the modification of the difference in pressure between the first pressure and the second pressure for the subsequent sleep session portion, (iii) the modification of the rate of change from the first pressure to the second pressure for the subsequent sleep session portion, or (iv) any combination of (i)-(iii), is based only on the data associated with the current sleep session, and the data associated with the second set of one or more prior sleep sessions.

13. The system of claim 12, wherein:

the data associated with the one or more prior sleep sessions is indicative of activities undertaken by the user before each of the one or more prior sleep sessions; or
the data associated with the one or more prior sleep sessions is indicative of activities undertaken by the user before each of the one or more prior sleep sessions, the activities including (i) consumption of alcohol, (ii) consumption of food, (iii) consumption of caffeine, or (iv) any combination of (i)-(iii).

14. The system of any one of claims 1 to 13, wherein:

the one or more processors of the control system are further configured to execute the machine-readable instructions to receive data associated with activities undertaken by the user before the current sleep session, and wherein (i) the modification of the first pressure for the subsequent sleep session portion, (ii) the modification of the difference in pressure between the first pressure and the second pressure for the subsequent sleep session portion, (iii) the modification of the rate of change from the first pressure to the second pressure for the subsequent sleep session portion, or (iv) any combination of (i)-(iii), is based at least in part on the activities undertaken by the user before the current sleep session; and/or

(a) the one or more processors of the control system are further configured to execute the machine-readable instructions to transmit, in response to the number of events experienced during the first portion of the current sleep session satisfying the threshold, a notification to the user or to a third party; or
(b) the one or more processors of the control system are further configured to execute the machine-readable instructions to transmit, in response to the number of events experienced during the first portion of the current sleep session satisfying the threshold, a notification to the user or to a third party, the third party including a healthcare provider of the user, a family member of the user, a friend of the user, a caregiver of the user, or any combination thereof.

15. The system of any one of claims 1 to 14, wherein:

the predetermined range of pressures is a range of pressures prescribed by a healthcare provider of the user; and/or
the number of events and the type of each event is determined based on pressure data associated with of a pressure of the pressurized air, and flow rate data associated with a flow rate of the pressurized air; and/or
the events include one or more obstructive apneas, one or more hypopneas, one or more central apneas, one or more mixed apneas, one or more snores, one or more flow limitations, or any combination thereof.

16. The system of any one of claims 1 to 15, wherein:

the one or more processors of the control system are further configured to execute the machine-readable instructions to determine a body position of the user during the subsequent sleep session portion, and wherein in response to the threshold being satisfied and the user being in a first body position, no modifications are made for the subsequent sleep session portion; or
the one or more processors of the control system are further configured to execute the machine-readable instructions to determine a body position of the user during the subsequent sleep session portion, and wherein in response to the threshold being satisfied and the user being in a first body position, no modifications are made for the subsequent sleep session portion, and wherein in response to the threshold being satisfied and the user being in a second body position, one or more of the modifications are modify for the subsequent sleep session portion.

17. The system of any one of claims 1 to 16, wherein;

the one or more processors of the control system are further configured to execute the machine-readable instructions to determine a sleep stage of the user during the subsequent sleep session portion, and wherein in response to the threshold being satisfied and the user being in a first sleep stage, no modifications are made for the subsequent sleep session portion; or
the one or more processors of the control system are further configured to execute the machine-readable instructions to determine a sleep stage of the user during the subsequent sleep session portion, and wherein in response to the threshold being satisfied and the user being in a first sleep stage, no modifications are made for the subsequent sleep session portion, and wherein in response to the threshold being satisfied and the user being in a second sleep stage, one or more of the modifications are modify for the subsequent sleep session portion.

**Patentansprüche**

1. System (100) umfassend:

ein Atemtherapiesystem (120), das so konfiguriert ist, dass es einem Benutzer (210) während einer aktuellen Schlafsitzung über das Atemtherapiesystem (120) Druckluft mit einem ersten Druck aus einem vorbestimmten Druckbereich zuführt, wobei der vorbestimmte Druckbereich mindestens einen zweiten Druck umfasst, der größer ist als der erste Druck;

einen Speicher (114), der maschinenlesbare Anweisungen speichert; und

ein Steuersystem (110) mit einem oder mehreren Prozessoren (112), die so konfiguriert sind, dass sie die maschinenlesbaren Befehle ausführen, um:

eine Anzahl von Ereignissen zu bestimmen (504), die der Benutzer während eines ersten Abschnitts der aktuellen Schlafsitzung erlebt, während die Druckluft mit dem ersten Druck zugeführt wird, einen Typ jedes Ereignisses zu bestimmen, das der Benutzer während des ersten Abschnitts der aktuellen Schlafsitzung erlebt, oder beides; und

als Reaktion darauf, dass die Anzahl der Ereignisse, die Art jedes Ereignisses oder beides, die während des ersten Abschnitts der aktuellen Schlafsitzung aufgetreten sind, einen Schwellenwert erreichen, (i) den ersten Druck so zu modifizieren (506), dass er ein modifizierter erster Druck für einen nachfolgenden Abschnitt der Schlafsitzung des Benutzers ist, sodass zu Beginn des nachfolgenden Abschnitts der Schlafsitzung die Druckluft über das Atemtherapiesystem (120) mit dem modifizierten ersten Druck an den Benutzer abgegeben wird, (ii) eine Druckdifferenz zwischen dem ersten Druck und dem zweiten Druck des vorbestimmten Druckbereichs für den nachfolgenden Schlafsitzungsabschnitt, (iii) eine Änderungsrate vom ersten Druck zum zweiten Druck des vorbestimmten Druckbereichs für den nachfolgenden Schlafsitzungsabschnitt oder (iv) eine beliebige Kombination aus (i) bis (iii)

wobei während der aktuellen Schlafsitzung ein Druck der zugeführten Druckluft nach einer ersten Zeitspanne beginnt, von dem ersten Druck auf einen zweiten Druck anzusteigen, **dadurch gekennzeichnet, dass** während der aktuellen Schlafsitzung eine zweite Zeitspanne zwischen (i) dem Beginn des Anstiegs des Drucks der zugeführten Druckluft von dem ersten Druck und (ii) dem Erreichen eines zweiten Drucks durch den Druck der zugeführten Druckluft verstreicht, und

wobei das Modifizieren der Änderungsrate vom ersten Druck zum zweiten Druck für den nachfolgenden Schlafsitzungsabschnitt das Modifizieren der zweiten Zeitspanne für den nachfolgenden Schlafsitzungsabschnitt umfasst, um eine modifizierte zweite Zeitspanne zu erhalten.

2. System nach Anspruch 1, wobei:

(a) der nachfolgende Schlafabschnitt einen oder mehrere nachfolgende Abschnitte des aktuellen Schlafabschnitts, einen oder mehrere Abschnitte eines nachfolgenden Schlafabschnitts oder beides umfasst und/oder der Beginn des nachfolgenden Schlafabschnitts eintritt, wenn der Benutzer während des nachfolgenden Schlafabschnitts zunächst einschläft, wenn der erste Druck im vorbestimmten Druckbereich angelegt wird oder beides; oder

(b) der modifizierte erste Druck des nachfolgenden Schlafabschnitts größer ist als der erste Druck des aktuellen Schlafabschnitts.

3. System nach Anspruch 1 oder Anspruch 2, wobei:

der eine oder die mehreren Prozessoren des Steuersystems ferner so konfiguriert sind, dass sie die maschinenlesbaren Anweisungen ausführen, um einen Mindestdruck zu bestimmen, bei dem eine durchschnittliche Anzahl von Ereignissen für einen vorbestimmten Zeitraum während des ersten Abschnitts der aktuellen Schlafsitzung den Schwellenwert nicht erfüllt, wobei der modifizierte erste Druck des nachfolgenden Schlafsitzungsabschnitts gleich dem Mindestdruck ist; oder

der eine oder die mehreren Prozessoren des Steuersystems ferner so konfiguriert sind, dass sie die maschinenlesbaren Anweisungen ausführen, um einen Mindestdruck zu bestimmen, bei dem eine durchschnittliche Anzahl von Ereignissen für einen vorbestimmten Zeitraum während des ersten Abschnitts der aktuellen Schlafsitzung den Schwellenwert nicht erfüllt, wobei der modifizierte erste Druck des nachfolgenden Schlafsitzungsabschnitts dem Mindestdruck entspricht; und wobei (i) der Mindestdruck größer ist als der erste Druck der aktuellen Schlafsitzung, (ii) der vorbestimmte Zeitraum eine Stunde beträgt; oder (iii) sowohl (i) als auch (ii) zutreffen.

4. System nach einem der Ansprüche 1 bis 3, wobei:

das Modifizieren der Druckdifferenz zwischen dem ersten Druck und dem zweiten Druck für den nachfolgenden Schlafsitzungsabschnitt das Modifizieren des zweiten Drucks zu einem modifizierten zweiten Druck umfasst; und/oder

das Modifizieren der Druckdifferenz zwischen dem ersten Druck und dem zweiten Druck für den nachfolgenden Schlafabschnitt das Modifizieren des zweiten Drucks zu einem modifizierten zweiten Druck umfasst, der größer ist als der erste Druck, der zweite Druck oder beide.

5. System nach einem der Ansprüche 1 bis 4, wobei:

das Modifizieren der Änderungsrate vom ersten Druck zum zweiten Druck für den nachfolgenden Schlafabschnitt das Modifizieren der ersten Zeitspanne für den nachfolgenden Schlafabschnitt zu einer modifizierten ersten Zeitspanne umfasst; oder

das Modifizieren der Änderungsrate vom ersten Druck zum zweiten Druck für den nachfolgenden Schlafabschnitt das Modifizieren der ersten Zeitspanne für den nachfolgenden Schlafabschnitt zu einer modifizierten ersten Zeitspanne umfasst, die kürzer ist als die erste Zeitspanne für den aktuellen Schlafabschnitt; oder

das Modifizieren der Änderungsrate vom ersten Druck zum zweiten Druck für den nachfolgenden Schlafabschnitt das Modifizieren der ersten Zeitspanne für den nachfolgenden Schlafabschnitt umfasst, sodass diese zu einer modifizierten ersten Zeitspanne wird, die kürzer ist als die erste Zeitspanne für den aktuellen Schlafabschnitt, wobei der Druck der zugeführten Druckluft im nachfolgenden Schlafabschnitt so konfiguriert ist, dass er nach der modifizierten ersten Zeitspanne vom ersten Druck zum zweiten Druck ansteigt.

6. System nach einem der Ansprüche 1 bis 5, wobei:

der Druck während der aktuellen Schlafsitzung so konfiguriert ist, dass er von dem ersten Druck auf den zweiten Druck ansteigt, nachdem eine Schwellenanzahl von Ereignissen während des ersten Abschnitts der aktuellen Schlafsitzung aufgetreten ist, in dem dem Benutzer die Druckluft mit dem ersten Druck zugeführt wird; oder

der Druck während der aktuellen Schlafsitzung so konfiguriert ist, dass er nach einer Schwellenanzahl von Ereignissen, die während des ersten Abschnitts der aktuellen Schlafsitzung auftreten, bei der dem Benutzer Druckluft mit dem ersten Druck zugeführt wird, vom ersten Druck auf den zweiten Druck anzusteigen beginnt, und wobei das Modifizieren der Änderungsrate vom ersten Druck zum zweiten Druck für den nachfolgenden Schlafabschnitt das Modifizieren der Schwellenanzahl von Ereignissen zu einer modifizierten Schwellenanzahl von Ereignissen für den nachfolgenden Schlafabschnitt umfasst.

7. System nach Anspruch 6, wobei:

die modifizierte Schwellenwertanzahl von Ereignissen für den nachfolgenden Schlafsitzungsabschnitt geringer ist als die Schwellenwertanzahl von Ereignissen für die aktuelle Schlafsitzung; oder

die modifizierte Schwellenanzahl von Ereignissen für den nachfolgenden Schlafabschnitt kleiner ist als die Schwellenanzahl von Ereignissen für die aktuelle Schlafphase, und wobei der Druck während des nachfolgenden Schlafabschnitts so konfiguriert ist, dass er nach einer modifizierten ersten Zeitspanne, die kürzer ist als die erste Zeitspanne der aktuellen Schlafphase, vom ersten Druck auf den zweiten Druck ansteigt.

8. System nach einem der Ansprüche 1 bis 7, wobei:

die modifizierte zweite Zeitspanne für den nachfolgenden Schlafsitzungsabschnitt kürzer ist als die zweite Zeitspanne für die aktuelle Schlafsitzung; oder

die modifizierte zweite Zeitspanne für den nachfolgenden Schlafabschnitt kürzer ist als die zweite Zeitspanne für die aktuelle Schlafphase, und wobei während des nachfolgenden Schlafabschnitts die modifizierte zweite Zeitspanne zwischen (i) dem Beginn des Anstiegs des Drucks vom ersten Druck oder dem modifizierten ersten Druck und (ii) dem Erreichen des zweiten Drucks oder des modifizierten zweiten Drucks vergeht.

9. System nach einem der Ansprüche 1 bis 8, wobei:

der Schwellenwert ein durchschnittlicher Schwellenwert der Anzahl von Ereignissen pro Zeiteinheit für den ersten Abschnitt der aktuellen Schlafsitzung und/oder für mehrere Abschnitte der aktuellen Schlafsitzung ist; oder

der Schwellenwert ein Schwellenwert für die durchschnittliche Anzahl von Ereignissen pro Zeiteinheit für den ersten Teil der aktuellen Schlafsitzung und/oder für mehrere Teile der aktuellen Schlafsitzung ist, und der Schwellenwert für die durchschnittliche Anzahl von Ereignissen pro Zeiteinheit auf der Grundlage des Drucks der

33

Druckluft während jedes Ereignisses, einer Körperposition des Benutzers während jedes Ereignisses, einer Schlafphase des Benutzers während jedes Ereignisses oder einer beliebigen Kombination davon gewichtet wird; oder

der Schwellenwert eine durchschnittliche Schwellenanzahl von Ereignissen pro Zeiteinheit für den ersten Abschnitt der aktuellen Schlafsitzung ist; und/oder für mehrere Abschnitte der aktuellen Schlafsitzung, und die Schwellenwert-Durchschnittszahl von Ereignissen pro Zeiteinheit wird auf der Grundlage des Drucks der Druckluft während jedes Ereignisses, einer Körperposition des Benutzers während jedes Ereignisses, einer Schlafphase des Benutzers während jedes Ereignisses oder einer beliebigen Kombination davon gewichtet, und wobei Ereignisse, die auftreten, wenn die Druckluft mit dem zweiten Druck zugeführt wird, stärker gewichtet werden als Ereignisse, die auftreten, wenn die Druckluft mit dem ersten Druck zugeführt wird.

10. System nach einem der Ansprüche 1 bis 9, wobei der Schwellenwert eine durchschnittliche Schwellenanzahl von Ereignissen pro Zeiteinheit ist, während der Benutzer während der aktuellen Schlafsitzung schläft.

11. System nach einem der Ansprüche 1 bis 10, wobei der eine oder die mehreren Prozessoren des Steuersystems ferner so konfiguriert sind, dass sie die maschinenlesbaren Anweisungen ausführen, um Daten zu empfangen, die mit der aktuellen Schlafsitzung und Daten verbunden sind, die mit einer oder mehreren früheren Schlafsitzungen des Benutzers verbunden sind, und wobei (i) die Änderung des ersten Drucks für den nachfolgenden Teil der Schlafsitzung, (ii) die Änderung der Druckdifferenz zwischen dem ersten Druck und dem zweiten Druck für den nachfolgenden Schlafabschnitt, (iii) die Änderung der Änderungsrate vom ersten Druck zum zweiten Druck für den nachfolgenden Schlafabschnitt oder (iv) eine beliebige Kombination aus (i) bis (iii) zumindest teilweise auf den Daten basiert, die mit einer oder mehreren vorherigen Schlafsitzungen verbunden sind.

12. System nach Anspruch 11, wobei der eine oder die mehreren Prozessoren des Steuersystems ferner so konfiguriert sind, dass sie die maschinenlesbaren Anweisungen ausführen, um:

einen ersten Satz einer oder mehrerer vorherigen Schlafsitzungen der einen oder mehreren vorherigen Schlafsitzungen zu identifizieren;
einen zweiten Satz einer oder mehrerer vorheriger Schlafsitzungen der einen oder mehreren vorherigen Schlafsitzungen zu identifizieren; und
die mit dem ersten Satz einer oder mehrerer vorheriger Schlafsitzungen verbundenen Daten zu verwerfen, so dass (i) die Änderung des ersten Drucks für den nachfolgenden Schlafsitzungsabschnitt, (ii) die Änderung der Druckdifferenz zwischen dem ersten Druck und dem zweiten Druck für den nachfolgenden Schlafsitzungsabschnitt, (iii) die Änderung der Änderungsrate vom ersten Druck zum zweiten Druck für den nachfolgenden Schlafsitzungsabschnitt oder (iv) eine beliebige Kombination aus (i) bis (iii) nicht auf den Daten basiert, die mit dem ersten Satz einer oder mehrerer vorheriger Schlafsitzungen verbunden sind; oder
wobei der eine oder die mehreren Prozessoren des Steuersystems ferner so konfiguriert sind, dass sie die maschinenlesbaren Anweisungen ausführen, um:

einen ersten Satz einer oder mehrerer vorherigen Schlafsitzungen der einen oder mehreren vorherigen Schlafsitzungen zu identifizieren;
einen zweiten Satz einer oder mehrerer vorheriger Schlafsitzungen der einen oder mehreren vorherigen Schlafsitzungen zu identifizieren; und
die mit dem ersten Satz einer oder mehrerer vorheriger Schlafsitzungen verbundenen Daten zu verwerfen, so dass (i) die Änderung des ersten Drucks für den nachfolgenden Schlafsitzungsabschnitt, (ii) die Änderung der Druckdifferenz zwischen dem ersten Druck und dem zweiten Druck für den nachfolgenden Schlafsitzungsabschnitt, (iii) die Änderung der Änderungsrate vom ersten Druck zum zweiten Druck für den nachfolgenden Schlafsitzungsabschnitt oder (iv) eine beliebige Kombination aus (i) bis (iii) nur auf den Daten basieren, die mit der aktuellen Schlafsitzung verbunden sind, und den Daten, die mit dem zweiten Satz einer oder mehrerer vorheriger Schlafsitzungen verbunden sind.

13. System nach Anspruch 12, wobei:

die Daten, die mit der einen oder den mehreren vorherigen Schlafsitzungen verbunden sind, Aktivitäten anzeigen, die der Benutzer vor jeder der einen oder mehreren vorherigen Schlafsitzungen durchgeführt hat; oder
die Daten, die mit der einen oder den mehreren vorherigen Schlafsitzungen verbunden sind, Aktivitäten anzeigen, die der Benutzer vor jeder der einen oder mehreren vorherigen Schlafsitzungen durchgeführt hat, wobei die Aktivitäten (i) den Konsum von Alkohol, (ii) den Konsum von Nahrungsmitteln, (iii) den Konsum von Koffein oder (iv) eine beliebige Kombination aus (i) bis (iii) umfassen.

14. System nach einem der Ansprüche 1 bis 13, wobei:
der eine oder die mehreren Prozessoren des Steuersystems ferner so konfiguriert sind, dass sie die maschinenlesbaren Anweisungen ausführen, um Daten zu empfangen, die mit Aktivitäten verbunden sind, die der Benutzer vor der aktuellen Schlafsitzung durchgeführt hat, und wobei (i) die Änderung des ersten Drucks für den nachfolgenden Schlafsitzungsabschnitt, (ii) die Änderung der Druckdifferenz zwischen dem ersten Druck und dem zweiten Druck für den nachfolgenden Schlafsitzungsabschnitt, (iii) die Änderung der Änderungsrate vom ersten Druck zum zweiten Druck für den nachfolgenden Schlafabschnitt oder (iv) eine beliebige Kombination aus (i) bis (iii) zumindest teilweise auf den Aktivitäten basieren, die der Benutzer vor der aktuellen Schlafsitzung durchgeführt hat; und/oder

(a) der eine oder die mehreren Prozessoren des Steuersystems sind ferner so konfiguriert, dass sie die maschinenlesbaren Befehle ausführen, um als Reaktion auf die Anzahl der Ereignisse, die während des ersten Abschnitts der aktuellen Schlafsitzung aufgetreten sind und den Schwellenwert erfüllen, eine Benachrichtigung an den Benutzer oder einen Dritten zu senden; oder
(b) der eine oder die mehreren Prozessoren des Steuerungssystems sind ferner so konfiguriert, dass sie die maschinenlesbaren Anweisungen ausführen, um als Reaktion auf die Anzahl der Ereignisse, die während des ersten Abschnitts der aktuellen Schlafsitzung aufgetreten sind und den Schwellenwert erfüllen, eine Benachrichtigung an den Benutzer oder an einen Dritten zu senden, wobei der Dritte einen Gesundheitsdienstleister des Benutzers, ein Familienmitglied des Benutzers, einen Freund des Benutzers, eine Pflegekraft des Benutzers oder eine beliebige Kombination davon umfasst.

15. System nach einem der Ansprüche 1 bis 14, wobei:

der vorbestimmte Druckbereich ein von einem Gesundheitsdienstleister des Benutzers vorgeschriebener Druckbereich ist; und/oder
die Anzahl der Ereignisse und die Art jedes Ereignisses auf der Grundlage von Druckdaten, die mit einem Druck der Druckluft verbunden sind, und Durchflussraten-Daten, die mit einer Durchflussrate der Druckluft verbunden sind, bestimmt werden; und/oder
die Ereignisse eine oder mehrere obstruktive Apnoen, eine oder mehrere Hypopnoen, eine oder mehrere zentrale Apnoen, eine oder mehrere gemischte Apnoen, ein oder mehrere Schnarchen, eine oder mehrere Durchflussbegrenzungen oder eine beliebige Kombination davon umfassen.

16. System nach einem der Ansprüche 1 bis 15, wobei:

der eine oder die mehreren Prozessoren des Steuersystems ferner so konfiguriert sind, dass sie die maschinenlesbaren Anweisungen ausführen, um eine Körperposition des Benutzers während des nachfolgenden Schlafabschnitts zu bestimmen, und wobei als Reaktion darauf, dass der Schwellenwert erreicht ist und sich der Benutzer in einer ersten Körperposition befindet, keine Änderungen für den nachfolgenden Schlafabschnitt vorgenommen werden; oder
der eine oder die mehreren Prozessoren des Steuersystems ferner so konfiguriert sind, dass sie die maschinenlesbaren Anweisungen ausführen, um eine Körperposition des Benutzers während des nachfolgenden Schlafabschnitts zu bestimmen, und wobei als Reaktion darauf, dass der Schwellenwert erreicht ist und sich der Benutzer in einer ersten Körperposition befindet, keine Änderungen für den nachfolgenden Schlafabschnitt vorgenommen werden, und wobei als Reaktion darauf, dass der Schwellenwert erreicht ist und sich der Benutzer in einer zweiten Körperposition befindet, eine oder mehrere der Änderungen für den nachfolgenden Schlafabschnitt vorgenommen werden.

17. System nach einem der Ansprüche 1 bis 16, wobei

der eine oder die mehreren Prozessoren des Steuersystems ferner so konfiguriert sind, dass sie die maschinenlesbaren Anweisungen ausführen, um eine Schlafphase des Benutzers während des nachfolgenden Schlafabschnitts zu bestimmen, und wobei als Reaktion darauf, dass der Schwellenwert erreicht ist und sich der Benutzer in einer ersten Schlafphase befindet, keine Änderungen für den nachfolgenden Schlafabschnitt vorgenommen werden; oder
der eine oder die mehreren Prozessoren des Steuersystems ferner so konfiguriert sind, dass sie die maschinenlesbaren Anweisungen ausführen, um eine Schlafphase des Benutzers während des nachfolgenden Schlafabschnitts zu bestimmen, und wobei als Reaktion darauf, dass der Schwellenwert erreicht ist und sich der Benutzer in einer ersten Schlafphase befindet, keine Änderungen für den nachfolgenden Schlafabschnitt vorgenommen werden, und wobei als Reaktion darauf, dass der Schwellenwert erreicht ist und sich der Benutzer in einer zweiten Schlafphase befindet, eine oder mehrere der Änderungen für den nachfolgenden Schlafabschnitt vorgenommen werden.

## Revendications

1. Système (100) comprenant :

un système de thérapie respiratoire (120) configuré pour fournir de l'air sous pression à une première pression d'une plage de pressions prédéterminée à un utilisateur (210) via le système de thérapie respiratoire (120) pendant une session de sommeil en cours, la plage de pressions prédéterminée comprenant en outre au moins une seconde pression supérieure à la première pression ;
une mémoire (114) stockant des instructions lisibles par machine ; et
un système de commande (110) comprenant un ou plusieurs processeurs (112) configurés pour exécuter les instructions lisibles par machine pour :

déterminer (504) un nombre d'événements subis par l'utilisateur pendant une première partie de la session de sommeil en cours pendant que l'air sous pression est fourni à la première pression, déterminant un type de chaque événement subi par l'utilisateur pendant la première partie de la session de sommeil en cours, ou les deux ; et
en réponse au nombre d'événements, au type de chaque événement, ou aux deux, subis pendant la première partie de la session de sommeil en cours satisfaisant à un seuil, modifier (506) (i) la première pression pour qu'elle soit une première pression modifiée pour une partie de session de sommeil ultérieure de l'utilisateur, de telle sorte qu'au début de la partie de session de sommeil ultérieure, l'air sous pression soit fourni à l'utilisateur via le système de thérapie respiratoire (120) à la première pression modifiée, (ii) une différence de pression entre la première pression et la seconde pression de la plage de pressions prédéterminée pour une partie de la session de sommeil ultérieure, (iii) un taux de variation de la première pression à la seconde pression de la plage de pressions prédéterminée pour une partie de la session de sommeil, ou (iv) toute combinaison de (i) à (iii),

dans lequel, pendant la session de sommeil en cours, une pression de l'air sous pression fourni commence à augmenter de la première pression à une seconde pression après une première période de temps, **caractérisé en ce que**, pendant la session de sommeil en cours, une seconde période de temps s'écoule entre (i) le moment où la pression de l'air sous pression fourni commence à augmenter de la première pression et (ii) le moment où la pression de l'air sous pression fourni atteint une seconde pression, et
dans lequel la modification du taux de variation entre la première pression et la seconde pression pour la partie de session de sommeil ultérieure comprend la modification de la seconde période de temps pour la partie de session de sommeil ultérieure afin qu'elle devienne une seconde période de temps modifiée.

2. Système selon la revendication 1, dans lequel :

(a) la partie de session de sommeil ultérieure comprend une ou plusieurs parties ultérieures de la session de sommeil en cours, une ou plusieurs parties d'une session de sommeil ultérieure, ou les deux, et/ou le début de la partie de session de sommeil ultérieure se produit lorsque l'utilisateur s'endort initialement pendant la partie de session de sommeil ultérieure, lorsque la première pression dans la plage de pressions prédéterminée est appliquée, ou les deux ; ou
(b) la première pression modifiée de la partie de la session de sommeil ultérieure est supérieure à la première pression de la session de sommeil en cours.

3. Système selon la revendication 1 ou 2, dans lequel :

le ou les processeurs du système de commande sont en outre configurés pour exécuter les instructions lisibles par machine pour déterminer une pression minimale à laquelle un nombre moyen d'événements pour une période de temps prédéterminée pendant la première partie de la session de sommeil actuelle ne satisfait pas au seuil, la première pression modifiée de la partie de session de sommeil ultérieure étant égale à la pression minimale ; ou

le ou les processeurs du système de commande sont en outre configurés pour exécuter les instructions lisibles par machine pour déterminer une pression minimale à laquelle un nombre moyen d'événements pour une période de temps prédéterminée pendant la première partie de la session de sommeil en cours ne satisfait pas au seuil, la première pression modifiée de la partie de session de sommeil ultérieure étant égale à la pression minimale ; et dans lequel (i) la pression minimale est supérieure à la première pression de la session de sommeil en cours, (ii) la période de temps prédéterminée est d'une heure ; ou (iii) à la fois (i) et (ii).

4. Système selon l'une quelconque des revendications 1 à 3, dans leque :

la modification de la différence de pression entre la première pression et la seconde pression pour la partie de session de sommeil ultérieure comprend la modification de la seconde pression pour qu'elle devienne une seconde pression modifiée ; et/ou
la modification de la différence de pression entre la première pression et la seconde pression pour la partie de session de sommeil ultérieure comprend la modification de la seconde pression pour qu'elle devienne une seconde pression modifiée qui est supérieure à la première pression, à la seconde pression, ou aux deux.

5. Système selon l'une quelconque des revendications 1 à 4, dans lequel :

la modification du taux de variation entre la première pression et la seconde pression pour la partie ultérieure de la session de sommeil comprend la modification de la première période de temps pour la partie ultérieure de la session de sommeil afin qu'elle devienne une première période de temps modifiée ; ou
la modification du taux de variation entre la première pression et la seconde pression pour la partie ultérieure de la session de sommeil comprend la modification de la première période de temps pour la partie ultérieure de la session de sommeil afin qu'elle devienne une première période de temps modifiée qui est inférieure à la première période de temps pour la session de sommeil en cours ; ou
la modification du taux de variation entre la première pression et la seconde pression pour la partie de session de sommeil ultérieure comprend la modification de la première période de temps pour la partie de session de sommeil ultérieure afin qu'elle devienne une première période de temps modifiée qui est inférieure à la première période de temps pour la session de sommeil en cours, la pression de l'air sous pression fourni dans la partie de session de sommeil ultérieure étant configurée pour augmenter de la première pression à la seconde pression après la première période de temps modifiée.

6. Système selon l'une quelconque des revendications 1 à 5, dans lequel :

la pression pendant la session de sommeil en cours est configurée pour commencer à augmenter de la première pression à la seconde pression après qu'un nombre seuil d'événements se soit produit pendant la première partie de la session de sommeil en cours, duquel l'air sous pression est fourni à l'utilisateur à la première pression ; ou
la pression pendant la session de sommeil en cours est configurée pour commencer à augmenter de la première pression à la seconde pression après qu'un nombre seuil d'événements se soit produit pendant la première partie de la session de sommeil en cours, à laquelle l'air sous pression est fourni à l'utilisateur à la première pression, et dans lequel la modification du taux de changement de la première pression à la seconde pression pour la partie ultérieure de la session de sommeil comprend la modification du nombre seuil d'événements pour qu'il devienne un nombre seuil modifié d'événements pour la partie ultérieure de la session de sommeil.

7. Système selon la revendication 6, dans lequel :

le nombre seuil modifié d'événements pour la partie de session de sommeil ultérieure est inférieur au nombre seuil d'événements pour la session de sommeil en cours ; ou
le nombre seuil modifié d'événements pour la partie de session de sommeil ultéireure est inférieur au nombre seuil d'événements pour la session de sommeil en cours, et dans lequel la pression pendant la partie de session de sommeil ultérieure est configurée pour augmenter de la première pression à la seconde pression après une première période de temps modifiée qui est inférieure à la première période de temps de la session de sommeil en cours.

8. Système selon l'une quelconque des revendications 1 à 7, dans lequel :

la seconde période de temps modifiée pour la partie de session de sommeil ultérieure est inférieure à la seconde période de temps pour la session de sommeil actuelle ; ou

la seconde période de temps modifiée pour la partie de session de sommeil ultérieure est inférieure à la seconde période de temps pour la session de sommeil en cours, et dans lequel, pendant la partie de session de sommeil ultérieure, la seconde période de temps modifiée s'écoule entre (i) le moment où la pression commence à augmenter à partir de la première pression ou de la première pression modifiée, et (ii) le moment où la pression atteint la seconde pression ou la seconde pression modifiée.

9. Système selon l'une quelconque des revendications 1 à 8, dans lequel :

le seuil est un nombre moyen de seuil d'événements par unité de temps pour la première partie de la session de sommeil en cours et/ou pour une pluralité de parties de la session de sommeil en cours ; ou
le seuil est un nombre moyen de seuil d'événements par unité de temps pour la première partie de la session de sommeil en cours ; et/ou pour une pluralité de parties de la session de sommeil en cours, et le nombre moyen de seuil d'événements par unité de temps est pondéré en fonction de la pression de l'air sous pression pendant chaque événement, de la position du corps de l'utilisateur pendant chaque événement, du stade de sommeil de l'utilisateur pendant chaque événement, ou de toute combinaison de ceux-ci ; ou
le seuil est un nombre moyen de seuil d'événements par unité de temps pour la première partie de la session de sommeil en cours ; et/ou pour une pluralité de parties de la session de sommeil en cours, et le nombre moyen de seuil d'événements par unité de temps est pondéré en fonction de la pression de l'air sous pression pendant chaque événement, de la position du corps de l'utilisateur pendant chaque événement, du stade de sommeil de l'utilisateur pendant chaque événement, ou de toute combinaison de ceux-ci, et dans lequel les événements se produisant lorsque l'air sous pression est fourni à la seconde pression sont pondérés plus lourdement que les événements se produisant lorsque l'air sous pression est fourni à la première pression.

10. Système selon l'une quelconque des revendications 1 à 9, dans lequel le seuil est un nombre moyen de seuil d'événements par unité de temps pendant que l'utilisateur est endormi au cours de la session de sommeil en cours.

11. Système selon l'une quelconque des revendications 1 à 10, dans lequel le ou les processeurs du système de commande sont en outre configurés pour exécuter les instructions lisibles par machine pour recevoir des données associées à la session de sommeil en cours et des données associées à une ou plusieurs sessions de sommeil ultérieures de l'utilisateur, et dans lequel (i) la modification de la première pression pour la partie de session de sommeil ultérieure, (ii) la modification de la différence de pression entre la première pression et la seconde pression pour la partie de session de sommeil ultérieure, (iii) la modification du taux de changement de la première pression et la seconde pression pour la partie de session de sommeil ultérieure, ou (iv) toute combinaison de (i) à (iii), est basée au moins en partie sur les données associées à la ou aux sessions de sommeil précédentes.

12. Système selon la revendication 11, dans lequel le ou les processeurs du système de commande sont en outre configurés pour exécuter les instructions lisibles par machine pour :

identifier un premier ensemble d'une ou plusieurs sessions de sommeil précédentes parmi la ou les sessions de sommeil précédentes ;
identifier un second ensemble d'une ou plusieurs sessions de sommeil précédentes parmi les une ou plusieurs sessions de sommeil précédentes ; et
supprimer les données associées au premier ensemble d'une ou plusieurs sessions de sommeil ultérieures, de telle sorte que (i) la modification de la première pression pour la partie de session de sommeil ultérieure, (ii) la modification de la différence de pression entre la première pression et la seconde pression pour la partie de session de sommeil ultérieure, (iii) la modification du taux de changement entre la première pression et la seconde pression pour la partie de session de sommeil ultérieure, ou (iv) toute combinaison de (i) à (iii), ne soient pas basées sur les données associées au premier ensemble d'une ou plusieurs sessions de sommeil précédentes ; ou
dans lequel le ou les processeurs du système de commande sont en outre configurés pour exécuter les instructions lisibles par machine pour :

identifier un premier ensemble d'une ou plusieurs sessions de sommeil précédentes parmi les une ou plusieurs sessions de sommeil précédentes ;
identifier un second ensemble d'une ou plusieurs sessions de sommeil antérieures parmi les une ou plusieurs sessions de sommeil antérieures ; et
supprimer les données associées au premier ensemble d'une ou plusieurs sessions de sommeil précédentes, de telle sorte que (i) la modification de la première pression pour la partie de session de sommeil ultérieure, (ii) la modification de la différence de pression entre la première pression et la seconde pression pour la partie de session de sommeil ultérieure, (iii) la modification du taux de changement entre la première pression et la seconde pression pour la partie de session de sommeil ultérieure, ou (iv) toute combinaison de (i) à (iii), soient basées uniquement sur les données associées à la session de sommeil en cours et les données associées au second ensemble d'une ou plusieurs sessions de sommeil précédentes.

**13.** Système selon la revendication 12, dans lequel :

les données associées avec la ou aux sessions de sommeil précédentes sont indicatives des activités entreprises par l'utilisateur avant chacune de la ou des sessions de sommeil précédentes ; ou
les données associées avec la ou aux sessions de sommeil précédentes indiquent les activités entreprises par l'utilisateur avant chacune de la ou des sessions de sommeil précédentes, les activités comprenant (i) la consommation d'alcool, (ii) la consommation de nourriture, (iii) la consommation de caféine, ou (iv) toute combinaison de (i) à (iii).

**14.** Système selon l'une quelconque des revendications 1 à 13, dans lequel :
le ou les processeurs du système de commande sont en outre configurés pour exécuter les instructions lisibles par machine pour recevoir des données associées aux activités entreprises par l'utilisateur avant la session de sommeil en cours, et dans lequel (i) la modification de la première pression pour la partie de session de sommeil ultérieure, (ii) la modification de la différence de pression et la première pression et la seconde pression pour la partie de session de sommeil ultérieure, (iii) la modification du taux de changement entre la première pression à la seconde pression pour la partie ultérieure de la session de sommeil, ou (iv) toute combinaison de (i) à (iii), sont basées au moins en partie sur les activités entreprises par l'utilisateur avant la session de sommeil en cours ; et/ou

(a) le ou les processeurs du système de commande sont en outre configurés pour exécuter les instructions lisibles par machine pour transmettre, en réponse au nombre d'événements survenus pendant la première partie de la session de sommeil en cours satisfaisant au seuil, une notification à l'utilisateur ou à un tiers ; ou
(b) le ou les processeurs du système de contrôle sont en outre configurés pour exécuter les instructions lisibles par machine pour transmettre, en réponse au nombre d'événements survenus pendant la première partie de la session de sommeil en cours satisfaisant au seuil, une notification à l'utilisateur ou à un tiers, le tiers comprenant un prestataire de soins de santé de l'utilisateur, un membre de la famille de l'utilisateur, un ami de l'utilisateur, un soignant de l'utilisateur, ou toute combinaison de ceux-ci.

**15.** Système selon l'une quelconque des revendications 1 à 14, dans lequel :

la plage de pressions prédéterminée est une plage de pressions prescrite par un prestataire de soins de santé de l'utilisateur ; et/ou
le nombre d'événements et le type de chaque événement sont déterminés sur la base de données de pression associées à une pression de l'air sous pression, et de données de débit associées à un débit de l'air sous pression ; et/ou
les événements comprennent une ou plusieurs apnées obstructives, une ou plusieurs hypopnées, une ou plusieurs apnées centrales, une ou plusieurs apnées mixtes, un ou plusieurs ronflements, une ou plusieurs limitations de débit, ou toute combinaison de ceux-ci.

**16.** Système selon l'une quelconque des revendications 1 à 15, dans lequel :

le ou les processeurs du système de commande sont en outre configurés pour exécuter les instructions lisibles par machine pour déterminer une position corporelle de l'utilisateur pendant la partie ultérieure de la session de sommeil, et dans lequel, en réponse au seuil étant satisfait et à l'utilisateur étant dans une première position corporelle, aucune modification n'est apportée pour la partie ultérieure de la session de sommeil ; ou
le ou les processeurs du système de commande sont en outre configurés pour exécuter les instructions lisibles par machine pour déterminer une position corporelle de l'utilisateur pendant la partie ultérieure de la session de sommeil, et dans lequel, en réponse au seuil satisfait et à l'utilisateur étant dans une première position corporelle, aucune modification n'est apportée à la partie ultérieure de la session de sommeil, et dans lequel, en réponse au seuil satisfait et à l'utilisateur étant dans une seconde position corporelle, une ou plusieurs des modifications sont modifiées à la partie ultérieure de la session de sommeil.

**17.** Système selon l'une quelconque des revendications 1 à 16, dans lequel :

le ou les processeurs du système de commande sont en outre configurés pour exécuter les instructions lisibles par machine pour déterminer un stade de sommeil de l'utilisateur pendant la partie de session de sommeil ultérieure, et dans lequel, en réponse au seuil satisfait et à l'utilisateur étant dans un premier stade de sommeil, aucune modification n'est apportée pour la partie de session de sommeil ultérieure ; ou

le ou les processeurs du système de contrôle sont en outre configurés pour exécuter les instructions lisibles par machine pour déterminer un stade de sommeil de l'utilisateur pendant la partie ultérieure de la session de sommeil, et dans lequel, en réponse au seuil satisfait et à l'utilisateur étant dans un premier stade de sommeil, aucune modification n'est apportée pour la partie ultérieure de la session de sommeil, et dans lequel, en réponse au seuil satisfait et à l'utilisateur étant dans un second stade de sommeil, une ou plusieurs des modifications sont modifiées pour la partie ultérieure de la session de sommeil.

**FIG. 1**

EP 4 329 848 B1

**FIG. 2**

EP 4 329 848 B1

**FIG. 3**

EP 4 329 848 B1

**FIG. 4**

EP 4 329 848 B1

**FIG. 5**

**FIG. 6A**

**FIG. 6B**

EP 4 329 848 B1

**FIG. 6C**

**FIG. 6D**

EP 4 329 848 B1

EP 4 329 848 B1

700

702

Supply pressurized air at
a plurality of pressures of
a predetermined range of
pressures

704

Determine a total amount of time that
the pressure of the pressurized air was
greater than a modification threshold
pressure

706A

Modify the first pressure
for a subsequent sleep
session portion

706B

Modify a difference between
the first pressure and a
second pressure for a
subsequent sleep session
portion

706C

Modify a rate of change
between the first
pressure and a second
pressure of a subsequent
sleep session portion

**FIG. 7**

**EP 4 329 848 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63181829
- US 2008097234 A1
- US 2015250963 A1
- US 2017274165 A1
- US 5245995 A
- WO 2008138040 A
- US 9358353 B
- WO 2016061629 A
- US 20170311879 A
- WO 2014047310 A
- US 10492720 B
- US 10660563 B
- US 20200337634 A
- WO 2017132726 A
- WO 2019122413 A
- US 20210150873 A
- WO 2019122414 A
- US 20200383580 A
- US 6502572 B
- WO 2018050913 A
- WO 2020104465 A